# EUROPÄISCHE PATENTSCHRIFT

(11) **EP 1 317 243 B1**
(45) Veröffentlichungstag und Bekanntmachung des Hinweises auf die Patenterteilung: **24.11.2004**
(21) Anmeldenummer: 01967329.2
(22) Anmeldetag: 11.09.2001
(51) Int. Cl.: A61K 7/13

(54) **HAARFÄRBEMITTEL MIT INDIGODERIVATEN**
HAIR DYEING AGENTS CONTAINING INDIGO DERIVATIVES
COLORANT CAPILLAIRE CONTENANT DES DERIVES D'INDIGO

(30) Priorität: 14.09.2000 DE 10045856
(43) Veröffentlichungstag der Anmeldung: 11.06.2003
(73) Patentinhaber: Henkel Kommanditgesellschaft auf Aktien, 40589 Düsseldorf-Holthausen (DE)
(72) Erfinder: HÖFFKES, Horst, 40595 Düsseldorf (DE); MÖLLER, Hinrich, 40789 Monheim (DE); NELLES, Karin, 40789 Monheim (DE)
(86) Internationale Anmeldenummer: PCT/EP2001/010480
(87) Internationale Veröffentlichungsnummer: WO 2002/022092

(56) Entgegenhaltungen:
- DE-C- 19 648 020
- FR-A- 2 787 708

## Beschreibung

Die vorliegende Erfindung betrifft Mittel zum Färben keratinischer Fasern, die spezielle Derivate des Benzo[b]furan-3-ons und/oder des Benzo[b]thiophen-3-ons sowie reaktive Verbindungen enthalten, ein Färbeverfahren mit dieser Farbstoffvorproduktkombination sowie die Verwendung dieser Kombination zum Färben keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintreten kann.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Ein weiteres neuartiges Färbesystem wird beispielsweise in der EP-A2-359 465 beschrieben. Im Rahmen dieses Verfahrens werden ausgewählte Isatinderivate mit aromatischen Aminen bei der Färbung keratinischer Fasern umgesetzt.

Dennoch wurde stets nach weiteren Färbesystemen gesucht, die intensive Färbungen der Fasern mit ausgezeichneten Echtheitseigenschaften ermöglichen.

Es wurde nunmehr überraschenderweise gefunden, daß spezielle Derivate des Benzo[b]furan-3-ons und/oder des Benzo[b]thiophen-3-ons mit speziellen reaktiven Komponenten zu intensiv gefärbten Verbindungen reagieren, die keratinische Fasern anfärben können.

Ein erster Gegenstand der vorliegenden Erfindung ist daher ein Mittel zur Färbung keratinischer Fasern, enthaltend in einem kosmetisch akzeptablen Medium als Farbstoffvorprodukte
(a) mindestens ein Derivat des Benzo[b]furan-3-ons und/oder mindestens ein Derivat des Benzo[b]thiophen-3-ons der Formel (1) 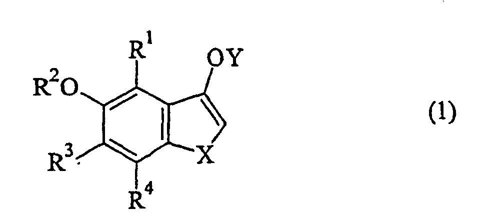 worin
   X steht für Schwefel oder Sauerstoff,
   Y steht für Wasserstoff, eine C₁₋₄-Alkylgruppe oder einen C₁₋₄-Acylrest,
   die Reste R¹, R³ und R⁴ stehen unabhängig voneinander für
   - Wasserstoff,
   - eine C₁₋₄-Alkylgruppe, die gegebenenfalls mit
      - einer oder mehreren Hydroxygruppe(n)
      - einer gegebenenfalls substituierten Aminogruppe,
      - einer gegebenenfalls mit einer C₁₋₄-Alkylgruppe veresterten Carboxygruppe oder
      - einer Gruppe -SO₂R⁵, wobei R⁵ steht für eine Hydroxygruppe, eine gegebenenfalls substituierte Aminogruppe oder eine C₁₋₄-Alkylgruppe
   substituiert sein kann,
   - eine gegebenenfalls substituierte Aminogruppe,
   - eine perfluorierte C₁₋₄-Alkylgruppe,
   - eine Cyangruppe,
   - eine C₂₋₅-Alkenylgruppe,
   - eine Nitrogruppe,
   - ein Halogenatom,
   - eine gegebenenfalls mit einer C₁₋₄-Alkylgruppe substituierte Mercaptogruppe,
   - eine Gruppe ―SO₂R⁵ oder
   - eine Gruppe ―OR⁶,
   - eine gegebenenfalls veresterte oder amidierte Carboxylgruppe und R² und gegebenenfalls R⁶ stehen unabhängig voneinander für
      - Wasserstoff,
      - eine C₁₋₄-Alkylgruppe, die gegebenenfalls mit
      - einer oder mehreren Hydroxygruppe(n),
      - einer gegebenenfalls substituierten Aminogruppe,
      - einer gegebenenfalls mit einer C₁₋₄-Alkylgruppe veresterten Carboxygruppe oder
      - einer Gruppe -SO₂R⁵
      substituiert sein kann, oder
   - eine perfluorierte C₁₋₄-Alkylgruppe,
      und
(b) mindestens eine reaktive Verbindung ausgewählt aus
   (b1) den reaktiven Carbonylverbindung, ausgewählt aus der Gruppe der aromatischen, heteroaromatischen oder ungesättigten Aldehyde oder Ketone, der Dialdehyde oder Diketone oder der Acetale, Halbaminale oder Iminderivate solcher reaktiver Carbonylverbindungen und/oder
   (b2) den CH-aktiven Verbindungen mit den Formeln (2) oder (3), 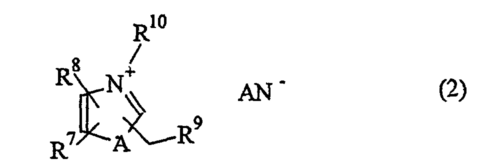 in der R¹⁰ steht für eine C₁₋₁₀-Alkylgruppe, eine C₂₋₄-Alkenylgruppe, eine C₂₋₄-Hydroxyalkylgruppe, eine C₂₋₄-Carboxyalkylgruppe, eine C₂₋₄-Sulfoalkylgruppe oder eine Aralkylgruppe,
      R⁷ und R⁸ unabhängig voneinander ein Wasserstoffatom, eine C₁₋₄-Alkylgruppe, ein Halogenatom, eine Hydroxygruppe, eine C₁₋₄-Alkoxygruppe oder eine Nitrogruppe bedeuten oder zusammen einen ankondensierten aromatischen Ring bilden,
      R⁹ steht für ein Wasserstoffatom, eine C₁₋₄-Alkylgruppe oder eine Arylgruppe,
      A steht für ein Sauerstoff- oder ein Schwefelatom, die Gruppe -CH=CHoder >N-R¹¹, in der R¹¹ steht für eine C₁₋₄-Alkylgruppe, eine C₂₋₄-Carboxyalkylgruppe, eine C₂₋₄-Sulfoalkylgruppe, eine C₂₋₄-Sulfoxyalkylgruppe, eine C₂₋₄-Hydroxyalkylgruppe oder eine Aralkylgruppe, und
      AN⁻ steht für ein Anion, das ausgewählt ist aus Halogenid, C₁₋₄-Alkylsulfat, C₁₋₄-Alkansulfonat, Arensulfonat, C₁₋₄-Perfluoralkansulfat, Tetrafluorborat, Perhalogenat, Sulfat, Hydrogensulfat oder Carboxylat, 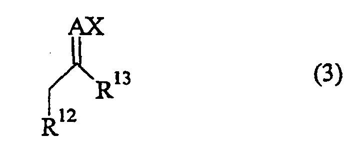 in der R¹² steht für eine C₁₋₄-Acylgruppe, eine C₁₋₄-Alkylsulfonylgruppe, eine C₁₋₄-Alkylsulfinylgruppe, eine C₁₋₄-Alkylaminogruppe, eine Di-C₁₋₄alkylaminogruppe, eine Vinylcarbonylgruppe, eine Methiniminogruppe, eine Nitrilgruppe, eine Ester- oder Carbonsäureamidgruppe, die gegebenenfalls durch eine C₁₋₄-Alkylgruppe, eine C₂₋₄-Hydroxyalkylgruppe oder eine Arylgruppe substituiert sein können, und R¹³ steht für eine C₁₋₄-Acylgruppe, eine C₁₋₄-Alkoxygruppe, eine C₁₋₄-Alkylaminogruppe, eine C₁₋₄-Acylaminogruppe oder eine Di-C₁₋₄-alkylaminogruppe, wobei die Reste R¹² und R¹³ auch gemeinsam mit dem Restmolekül ein 5-, 6- oder 7-gliedriges Ringsystem aus der Reihe der Thiazolidin-2,5-dione, Thiazolidin-2-thion-5-one, Perhydropyrimidin-2,4,6-trione, Perhydropyrimidin-2-thion-4,6-dione, Cyclopentan-1,3-dione, Cyclohexan-1,3-dione, Indan-1,3-dione, 2-Pyrazolin-5-one, 1,2-Dihydro-6-hydroxy-2-hydroxypyridine oder deren Enolester bilden können, und AX steht für Sauerstoff, Schwefel oder eine Dicyanmethylengruppe.

Unter keratinischen Fasern werden erfindungsgemäß Pelze, Wolle, Federn und insbesondere menschliche Haare verstanden.

Beispiele für die als Substituenten in den erfindungsgemäßen Verbindungen genannten C₁₋₄-Alkylgruppen sind die Gruppen Methyl, Ethyl, Propyl, Isopropyl und Butyl. Ethyl und Methyl sind bevorzugte Alkylgruppen. Bevorzugte C₁₋₄-Hydroxyalkylgruppen sind die Gruppen Hydroxymethyl, 2-Hydroxyethyl, 3-Hydroxypropyl oder 4-Hydroxybutyl; 2-Hydroxyethyl ist eine besonders bevorzugte Hydroxyalkylgruppe. Die weiteren verwendeten Begriffe leiten sich erfindungsgemäß von den hier gegebenen Definitionen ab. Eine erfindungsgemäß bevorzugte perfluorierte C₁₋₄-Alkylgruppe ist die Gruppe Trifluormethyl. Eine erfindungsgemäß bevorzugte Carboxy-C₁₋₄-alkylgruppe ist die Gruppe Carboxymethyl. Beispiele für ein Halogenatom sind erfindungsgemäß ein F-, ein Cl- oder ein Br-Atom, ein Cl-Atom ist besonders bevorzugt. Bevorzugte Amino-C₁₋₄alkylgruppen sind die Gruppen Aminomethyl, Aminoethyl, Diethylaminomethyl und Dimethylaminomethyl. Bevorzugte substituierte Aminogruppen sind die Amino-C₁₋₄alkylaminogruppen, die Aminoethylaminogruppe ist erfindungsgemäß von diesen besonders bevorzugt. Eine bevorzugte Acylgruppe ist die Acetylgruppe. Bevorzugte Alkenylgruppen sind die Vinylgruppe und die Allylgruppe.

Verbindungen der Formel (1), bei denen der Rest Y für Wasserstoff steht sind erfindungsgemäß besonders bevorzugt. Bei diese Substanzen, die in einem Keto-Enol-Gleichgewicht gemäß Formel (1a) 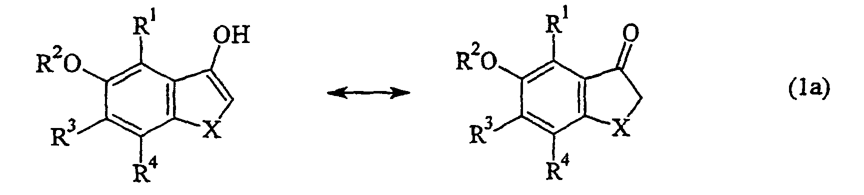 vorliegen, beziehen sich alle Aussagen der vorliegenden Anmeldung sowohl auf die Keto-Form dieser Verbindungen als auch auf die Enol-Form.

Als besonders vorteilhaft haben sich die Verbindungen der Formel (1) erwiesen, bei denen R¹ und/oder R⁴ für Wasserstoff stehen.

Weiterhin können auch die Verbindungen der Formel (1) bevorzugt sein, bei denen mindestens einer der Reste R¹, R³ oder R⁴ von Wasserstoff verschieden ist

Besonders bevorzugt ist der Rest -OR² ausgewählt aus einer Hydroxygruppe, einer Methoxygruppe, einer Carboxymethylgruppe und einer 2-Hydroxyethoxygruppe.

In einer ersten Variante der vorliegenden Erfindung sind Verbindungen der Formel (1) bevorzugt, bei denen X für ein Sauerstoffatom steht, wie beispielsweise die Verbindungen 5-Carboxymethoxy-benzo[b]furan-3-on, 5-Hydroxy-benzo[b]furan-3-on oder 5-(2'-Hydroxyethoxy)-benzo[b]furan-3-on.

In einer zweiten Variante der vorliegenden Erfindung sind Verbindungen bevorzugt, bei denen X für ein Schwefelatom steht, wie beispielsweise die Verbindungen 5-Hydroxybenzo[b]thiophen-3-on, 5-Carboxymethoxy-benzo[b]thiophen-3-on, 5,6-Dihydroxybenzo[b]thiophen-3-on oder 6-Hydroxy-5-methoxy-benzo[b]thiophen-3-on.

Erfindungsgemäß besonders bevorzugt sind Mittel, die mindestens eine Verbindung der Formel (1) und mindestens eine reaktive Carbonylverbindung, ausgewählt aus der Gruppe der aromatischen, heteroaromatischen oder ungesättigten Aldehyde oder Ketone, der Dialdehyde oder Diketone oder der Acetale, Halbaminale oder Iminderivate solcher reaktiver Carbonylverbindungen, enthalten.

Geeignete reaktive Carbonylverbindungen vom Typ der aromatischen Aldehyde sind z.B. in der deutschen Patentanmeldung DE 196 30 274 A1 und DE 196 30 275 A1 beschrieben. Weitere geeignete Arylaldehyde sind aus US 5,199,954 bekannt. Erfindungsgemäß geeignete Verbindungen sind insbesondere:
- Benzaldehyd und seine Derivate, wie beispielsweise 2-Hydroxybenzaldehyd, 4-Hydroxy-3-methoxy-benzaldehyd (Vanillin), 3-Hydroxy-4-methoxy-benzaldehyd (Isovanillin), 3,4-Dihydroxybenzaldehyd, 4-Hydroxybenzaldehyd, 3,5-Dimethoxy-4-hydroxybenzaldehyd, 4-Dimethylaminobenzaldehyd, 4-Hydroxy-2-methoxybenzaldehyd, 3,5-Dimethyl-4-hydroxybenzaldehyd, 4-Dimethylamino-2-methoxybenzaldehyd, 2-Hydroxy-3-methoxybenzaldehyd, 2,4-Dihydroxybenzaldehyd, 3,4-Dihydroxybenzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxybenzaldehyd, 2,5-Dimethoxybenzaldehyd, 3,5-Dimethoxybenzaldehyd, 3,4-Dimethoxybenzaldehyd, 4-Ethoxybenzaldehyd, 4-Carboxybenzaldehyd, 4-Diethylamino-3-methoxybenzaldehyd, 4-Diethylamino-2-hydroxybenzaldehyd, 4-Dibutylaminobenzaldehyd, 3,4-Dimethoxy-5-hydroxybenzaldehyd, 3-Ethoxy-4-hydroxybenzaldehyd, Biphenyl-4-carbaldehyd, 2-Nitrobenzaldehyd, 3-Nitrobenzaldehyd und 4-Nitrobenzaldehyd,
- 1-Naphthaldehyd und seine Derivate, wie beispielsweise 4-Hydroxy-1-naphthaldehyd, 4-Methoxy-1-naphthaldehyd, 4-Dimethylamino-1-naphthaldehyd sowie 2-Methoxy-1-naphthaldehyd und
- Zimtaldehyd und seine Derivate, wie beispielsweise 4-Hydroxy-3-methoxyzimtaldehyd (Coniferylaldehyd), 4-Dimethylaminozimtaldehyd und 3,5-Dimethoxy-4- hydroxyzimtaldehyd.
Besonders bevorzugt sind die Derivate der aromatischen Aldehyde, die in para-Stellung zu dem Substituenten, der die Aldehydfunktion trägt, eine gegebenenfalls substituierte Hydroxy- oder Aminogruppe trägt. Besonders bevorzugt ist hierbei die freie Hydroxygruppe sowie die Gruppe Di-(C₁₋₄-alkyl)-amino, wie beispielsweise die Gruppe Dimethylamino.

Geeignete reaktive Carbonylverbindungen vom Typ der heteroaromatischen Aldehyde sind beispielsweise in der deutschen Patentanmeldung DE 197 172 806 beschrieben. Besonders gut geeignete Farbstoffe sind beispielsweise trans-β-(2-Furyl)-acrolein, 1-Methylindol-3-carbaldehyd, 2-(1,3,3-Trimethyl-2-indolinyliden)-acetaldehyd, 4-Methylimidazol-5-carbaldehyd, 3-Methoxy-4-(1-pyrrolidinyl)-benzaldehyd, Pyrrol-2-carbaldehyd, Thiophen-2-carbaldehyd, Thiophen-3-carbaldehyd, Chromon-3-carbaldehyd, 6-Methyl-chromon-3-carbaldehyd, N-Methylpyrrol-2-carbaldehyd, 5-Methylfurfural, 6-Hydroxychromen-3-carbaldehyd, N-Ethylcarbazol-3-carbaldehyd, Indol-3-carbaldehyd, 6-Methylindol-3-carbaldehyd, Thiophen-2,3-dicarbaldehyd, Thiophen-2,5-dicarbaldehyd oder Antipyrin-4-carbaldehyd. Spezielle Produkte dieses Typs mit einer Pyridinium-Gruppe sind in der deutschen Patentanmeldung DE 197 45 356 beschrieben, beispielsweise die sehr gut geeigneten 4-Formyl-1-methylpyridiniumbenzolsulfonat und 4-Formyl-1-methylchinolinium-methan-sulfonat bzw. -methylsulfat bzw. -p-toluolsulfonat sowie das 2-Formyl-1-methylchinolinium-p-toluolsulfonat-hydrat.

Geeignete reaktive Carbonylverbindungen vom Typ der ungesättigten Aldehyde sind beispielsweise in der deutschen Patentanmeldung DE 197 17 224 beschrieben. Für die vorliegende Erfindung eignet sich besonders gut der Glutaconaldehyd in Form seiner Salze, beispielsweise seines Alkali- oder Tetrabutylammoniumsalzes oder der 2-Chlor-3-hydroxymethylen-1-cyclohexen-1-carbaldehyd. Ein weiterer erfindungsgemäß geeigneter ungesättigter Aldehyd ist das 5-(4-(Diethylamino)phenyl)-2,4-pentadienal.

Dialdehyde und Diketone und deren Derivate, die sich als reaktive Carbonylverbindungen erfindungsgemäß eignen, sind beispielsweise alicyclische und cyclische 1,2-, 1,3-oder 1,4-Dicarbonyl-verbindungen, wie Isatin, 1-H-Chinolin-2,3,4-trion, Ninhydrin, Alloxan, Isobarbitursäure, Benzol-1,4-dicarbaldehyd, 1,2-Phthaldialdehyd, 1,2-Cyclohexandion, 1,3-Cylcohexandion, 1,4-Cyclohexandion, Dimedon, 1,2-Cyclopentadion, 1,3-Cyclopentadion, p- und o-Chinone, 2-Methyl-1,4-naphthochinon, 1,3-Indandione und deren Derivate. Solche Farbstoffe finden sich beispielsweise in der deutschen Offenlegungsschrift DE 43 35 627 A1. Geeignete Verbindungen sind beispielsweise der Malondialdehyd, bevorzugt in Form seines Dimethylacetals, das 1,3-Indandion oder das 2-Acetyl-1,3-cyclohexandion. Eine geeignete 1,2-Dicarbonylverbindung ist auch das Isatinsäure-Kaliumsalz.

Zu den erfindungsgemäß als reaktive Carbonylverbindung geeigneten Diketonen gehören auch cyclische Dicarbonylverbindungen wie beispielsweise das Isatin und dessen Derivate, wie sie beispielsweise in der deutschen Offenlegungsschrift DE 44 09 143 A1 beschrieben sind. Erfindungsgemäß bevorzugt sind das Isatin, das Isatin-5-Sulfonsäure-Kaliumsalz, das N-Allylisatin, das 1-Piperidinomethylisatin, das 1-Hydroxymethylisatin und das 1-Diethylaminomethylisatin.

Eine weitere als reaktive Carbonylverbindung geeignete cyclische Dicarbonylverbindung ist beispielsweise auch die Dehydroascorbinsäure, deren Eignung als Haarfarbstoff aus der deutschen Patentanmeldung DE 197 45 354 bekannt ist.

Schließlich eignen sich erfindungsgemäß auch die Acetale, Iminderivate und Halbaminale der genannten reaktiven Carbonylverbindungen. Solche Verbindungen werden durch Reaktion der Carboxylgruppe mit primären Alkoholen oder Aminen und gegebenenfalls Wasserabspaltung erhalten.

Ausgehend von den ungesättigten Dialdehyden und Diketonen gelangt man dabei in die Gruppe der Merocyanin- und Cyanin- beziehungsweise Azomethin-Farbstoffe. Geeignete Imin-Derivate des Glutacondialdehyds sind beispielsweise das Mono-N-methylanilin-Derivat des Glutaconaldehyds (5-N-Methylanilinopentadienal) oder das N-(5-Anilino-2,4-pentadien-1-yliden)-anilinium-chlorid. Ein weiterer geeigneter vinyloger Cyaninfarbstoff ist das 7-Dimethylamino-2,4,6-heptatrienyliden-dimethylammoniumperchlorat. Solche Verbindungen sind als Haarfärbemittel-Komponenten beispielsweise aus der deutschen Patentanmeldung DE 197 17 223 bekannt.

Als Beispiele für bevorzugt eingesetzte Verbindungen der Formel (2) können genannt werden: 1,4-Dimethylchinolinium-, 1,2-Dimethylchinolinium-, 1,4-Dimethylpyridinium-, 1,2-Dimethylpyridinium-, 2,4,6-Trimethylpyrilium-, 2-Methyl-1-ethylchinolinium-, 2,3-Di-methylisochinolinium-, 1,2,3,3-Tetramethyl-3H-indolium-, 2,3-Dimethylbenzothiazolium-, 2,3-Dimethyl-6-nitrobenzothiazolium-, 3-Benzyl-2-benzothiazolium-, 2-Methyl-3-propylbenzothiazolium-, 2,4-Dimethyl-3-ethylthiazolium-, 3-(2-Carboxyethyl)-2,5-dimethylbenzothiazolium-, 1,2,3-Trimethylbenzimidazolium-, 5,6-Dichlor-1,3-diethyl-2-methylbenzimidazolium-, 3-Ethyl-2-methylbenzothiazolium-, 3-Ethyl-2-methylnaphtho[1,2-d]thiazolium-, 5-Chlor-3-ethyl-2-methylbenzothiazolium-, 3-Ethyl-2-methylbenzoxazolium-Salze, die z. B. als Chloride, Bromide, Iodide, Methansulfonate, Benzolsulfonate, p-Toluolsulfonate, Trifluormethansulfonate, Methylsulfate, Tetrafluorborate vorliegen können, sowie 2-Methyl-3-(3-sulfopropyl)-benzothiazoliumhydroxid-(inneres Salz), 4-Methyl-1-(3-sulfopropyl)-pyridinium-hydroxid-(inneres Salz), 4-Methyl-1-(3-sulfopropyl)-chinolinium-hydroxid-(inneres Salz), 5-Methoxy-2-methyl-3-(3-sulfopropyl)-benzothiazolium-hydroxid-(inneres Salz) und beliebige Gemische der voranstehenden.

Erfindungsgemäß können die Verbindungen der Formel (2) in Abhängigkeit des pH-Wert des jeweiligen Mittels auch in Form ihrer Basen beziehungsweise der Enamine vorliegen.

Beispiele für Verbindungen mit der Formel (3) sind Rhodamin, Rhodamin-3-essigsäure, Barbitursäure, Thiobarbitursäure, 1,3-Dimethyl-, 1,3-Diethylthiobarbitursäure, Oxindol, 3-Indoxylacetat, Cumaranon, 1-Methyl-3-phenylpyrazolinon, Indan-1,3-dion, Cyclopentan-1,3-dion, 1,2-Dihydro-1-ethyl-6-hydroxy-4-methyl-2-oxo-3-pyridincarbonitril, 1-Dicyanmethylenindan und beliebige Gemische der voranstehenden.

Die Vielfalt der erzielbaren Farbnuancen des erfindungsgemäßen Färbesystems kann durch Kombination mit einer oder mehreren Verbindungen mit primärer oder sekundärer Amino- oder Hydroxygruppe, ausgewählt aus der Gruppe der Aminosäuren und Peptide, der aromatischen Amine, Phenole, Aminophenole sowie stickstoffhaltigen Heterocyclen weiter erhöht werden. Dabei werden in vielen Fällen auch dunklere Nuancen erhalten.

Geeignete Aminosäuren sind insbesondere die natürlich vorkommenden und synthetischen Aminosäuren, beispielsweise Arginin, Histidin, Phenylalanin, Dihydroxyphenylalanin, Ornithin, Lysin. Geeignete Peptide sind vor allem Oligo- und Polypeptide, die eine ausreichende Wasserlöslichkeit in den erfindungsgemäßen Zubereitungen zur Keratinreduktion aufweisen. Als Beispiele sind Glutathion oder die in den Hydrolysaten von Kollagen, Keratin, Elastin, Casein oder Pflanzenproteinen, wie Sojaprotein, Weizenprotein, Algenprotein oder Mandelprotein, enthaltenen Oligopeptide zu nennen.

Geeignete aromatische Amine und Aminophenole sind N,N-Dimethyl-, N,N-Diethyl-, N-(2'-Hydroxyethyl)-N-ethyl-, N,N-Bis-(2'-hydroxyethyl)-, N-(2'-Methoxyethyl)-, 2-Chlor-, 2,3-, 2,4- und 2,5-Dichlor-p-phenylendiamin, 2,5-Dihydroxy-4-morpholinoanilindihydro-bromid, 2-, 3- und 4-Aminophenol, 2-Aminomethyl-4-aminophenol, 2-Hydroxymethyl-4-aminophenol, o- und p-Phenylendiamin, o- und m-Toluylendiamin, 2,5-Diamino-phenol, -toluol und -phenethol, 4-Amino-3-methylphenol, 2-(2',5'-Diaminophenyl)-ethanol, 2,4-Diaminophenoxy-ethanol, 2-(2',5'-Diaminophenoxy)-ethanol, 4-Methylamino-, 3-Amino-4-(2'-hydroxyethyloxy)-, 3,4-Methylendiamino- und 3,4-Methylendioxyanilin, 3-Amino-2,4-dichlor-, 4-Methylamino-, 2-Methyl-5-amino-, 3-Methyl-4-amino-, 2-Methyl-5-(2'hydro-xyethylamino)-, 6-Methyl-3-amino-2-chlor-, 2-Methyl-5-amino-4-chlor-, 3,4-Methylen-dioxy-, 5-(2'-Hydroxyethylamino)-4-methoxy-2-methyl- und 4-Amino-2-hydroxymethyl-phenol, 1,3-Diamino-2,4-dimethoxybenzol, 2-, 3-, 4-Aminobenzoesäure, - phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline beziehungsweise Phenole mit einem weiteren aromatischen Rest, wie sie in der Formel (4) dargestellt sind 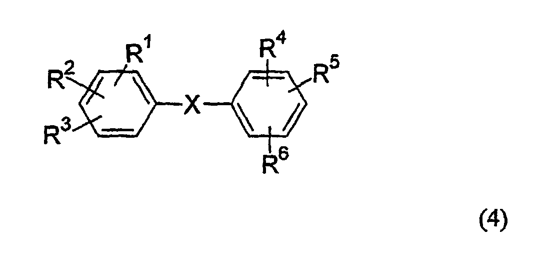 in der R¹ für eine Hydroxy- oder eine Aminogruppe, die durch C₁₋₄-Alkyl, C₁₋₄-Hydroxyalkyl- oder C₁₋₄-Alkoxy-C₁₋₄-alkyl substituiert sein kann, steht, R², R³, R⁴, R⁵ und R⁶ für Wasserstoff, eine Hydroxy- oder eine Aminogruppe, die durch C₁₋₄-Alkyl-, C₁₋₄-Hydroxyalkyl, C₁₋₄-Aminoalkyl- oder C₁₋₄-Alkoxy-C₁₋₄-alkylgruppe substituiert sein kann, für eine Carbon- oder Sulfonsäuregruppe stehen, und X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyloder Iminogruppe, ein Sauerstoff- oder Schwefelatom, oder eine Gruppe mit der Formel (5)

Z-(CH₂-Y-CH₂-Z')ₒ (5),

in der Y eine direkte Bindung, eine CH₂- oder CHOH-Gruppe bedeutet,
Z und Z' unabhängig voneinander ein Sauerstoffatom, eine NR⁷-Gruppe, worin R⁷ Wasserstoff, eine C₁₋₄-Alkyl- oder Hydroxy-C₁₋₄-alkylgruppe bedeutet, die Gruppe -O-(CH₂)ₚ-NH oder NH-(CH₂)ₚ-O, worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-monooder -di-Na-Salz, 4,4'-Diaminodiphenylmethan, -sulfid, -sulfoxid, -amin, 4,4'-Diaminodiphenyl-amin-2-sulfonsäure, 4,4'-Diaminobenzophenon, -diphenylether, 3,3',4,4'-Tetraaminodi-phenyl, 3,3',4,4'-Tetraamino-benzophenon, 1,3-Bis-(2',4'-diaminophenoxy)-propan, 1,8-Bis-(2',5'-diaminophenoxy)-3,6-dioxaoctan, 1,3-Bis-(4'-aminophenylamino)-propan, -2-propa-nol, 1,3-Bis-[N-(4'-aminophenyl)-2-hydroxyethylamino]-2-propanol, N,N-Bis-[2-(4'-amino-phenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salzoder Schwefelsäure, eingesetzt werden.

Geeignete Phenole sind beispielsweise das 2-, 3- oder 4-Methoxy-, das 3-Dimethylamino-, 2-(2'-Hydroxyethyl)- und das 3,4-Methylendioxy-phenol, das Resorcin und das 2-, 4-und 5-Methylresorcin, das 2- und 4-Chlorresorcin, 2,5-Dimethylresorcin, Brenzkatechin, Hydrochinon, Pyrogallol, Phloroglucin, Hydroxyhydrochinon, die 2,4- oder 3,4-Dihydroxybenzoe- oder -phenylessigsäure, die Gallussäure, die 2,4,6-Trihydroxybenzoesäure oder das 2,4,5-Trihydroxyacetophenon, das 1-Naphthol, das 1,5-, 2,3- und 2,7-Dihydroxynaphthalin, die 6-Dimethylamino-4-hydroxy-2-naphthalinsulfonsäure oder die 3,6-Dihy-droxy-2,7-naphthalindisulfonsäure.

Geeignete stickstoffhaltige heterocyclische Verbindungen sind beispielsweise 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino- und 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino- und 2-Amino-4-methoxy-6-methyl-pyrimidin, 3-Amino-, 3-Amino-5-hydroxy- und 3,5-Diaminopyrazol, 2-,3-, 8-Aminochinolin, 4-Amino-chinaldin, 2-, 6-Aminonicotinsäure, 5-Aminoisochinolin, 5-, 6-Aminoindazol, 5- und 7-Aminobenzimidazol und -benzothiazol, 2,5-Dihydroxy-4-morpholinoanilin sowie Indol- und Indolinderivate, wie 4-, 5-, 6- und 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, beispielsweise als Salze anorganischer Säuren, wie Salz- oder Schwefelsäure, eingesetzt werden.

Diese Färbesysteme können noch weiter verstärkt werden durch geeignete stickstoffhaltige Heterocyclen wie beispielsweise Piperidin, Piperidin-2-, -3- oder -4-carbonsäure, Pyridin, Pyridin-2-carbonsäure, Pyridin-3-carbonsäure, Pyridin-4-carbonsäure, 2-, 3- oder 4-Hydroxypyridin, Imidazol, 1-Methylimidazol, Histidin, Pyrrolidin, Pyrrolidon, Pyrrolidon-5-carbonsäure, Pyrazol, 1,2,4-Triazol, Piperazin sowie deren physiologisch verträglichen Salze.

In den erfindungsgemäßen Färbemittelzubereitungen können mehrere Farbstoffvorprodukte vom Typ der reaktiven Verbindungen (b) gleichzeitig enthalten sein. Desgleichen können auch mehrere Derivate des Benzo[b]furan-3-ons und/oder des Benzo[b]thiophen-3-ons in den erfindungsgemäßen Mitteln enthalten sein. Weiterhin können auch mehrere der weiteren Verbindungen aus der Gruppe der Aminosäuren, Peptide, aromatischen Amine, Aminophenole, Phenole und stickstoffhaltigen Heterocyclen gemeinsam enthalten sein, wenn dies zur Erzielung der gewünschten Farbnuance erforderlich ist.

Die Derivate des Benzo[b]furan-3-ons und/oder des Benzo[b]thiophen-3-ons sind in den erfindungsgemäßen Mitteln bevorzugt in Mengen von 0,02 - 8 Gew.-%, insbesondere von 0,05 - 5 Gew.-% und ganz besonders bevorzugt von 0,1 - 2 Gew.-%, jeweils bezogen auf die gesamte Anwendungszubereitung enthalten. Die reaktiven Verbindungen (b) sind in den erfindungsgemäßen Mitteln bevorzugt in Mengen von 0,02 - 8 Gew.-%, insbesondere von 0,05 - 5 Gew.-% und ganz besonders bevorzugt von 0,1 - 2 Gew.-%, jeweils bezogen auf die gesamte Anwendungszubereitung enthalten.

In einer weiteren Ausführungsform können die erfindungsgemäßen farbverändernden Mittel aber auch noch weitere Farbstoffe und/oder Farbstoffvorprodukte enthalten.

Hinsichtlich der in den erfindungsgemäßen Mitteln eingesetzbaren Farbstoffvorprodukten unterliegt die vorliegende Erfindung keinerlei Einschränkungen. Die erfindungsgemäßen Mittel können als Farbstoffvorprodukte
- Oxidationsfarbstoffvorprodukte vom Entwickler- und Kuppler-Typ, und
- Vorstufen naturanaloger Farbstoffe, wie Indol- und Indolin-Derivate,
sowie Mischungen von Vertretern dieser Gruppen enthalten.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (6) 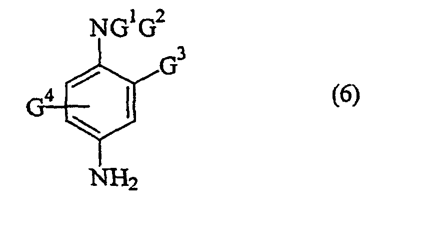 wobei
- G¹ steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein 4'-Aminophenylradikal oder ein C₁- bis C₄-Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal oder ein C₁- bis C₄-Alkylradikal, das mit einer stickstoffhaltigen Gruppe substituiert ist;
- G³ steht für ein Wasserstoffatom, ein Halogenatom, wie ein Chlor-, Brom, Jododer Fluoratom, ein C₁- bis C₄-Alkylradikal, ein C₁- bis C₄-Monohydroxyalkylradikal, ein C₁- bis C₄-Hydroxyalkoxyradikal, ein C₁- bis C₄-Acetylaminoalkoxyradikal, ein C₁- bis C₄- Mesylaminoalkoxyradikal oder ein C₁- bis C₄-Carbamoylaminoalkoxyradikal;
- G⁴ steht für ein Wasserstoffatom, ein Halogenatom oder ein C₁- bis C₄-Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyalkylgruppe ist besonders bevorzugt. Beispiele für Halogenatome sind erfindungsgemäß F-, Cl- oder Br-Atome, C1-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (6) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (6) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-pphenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-pphenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,-β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (6) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (7) entsprechen, sowie ihre physiologisch verträglichen Salze: 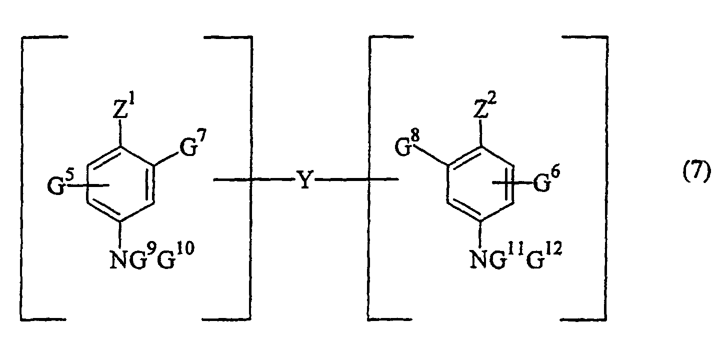 wobei:
- Z¹ und Z² stehen unabhängig voneinander für ein Hydroxyl- oder NH₂-Radikal, das gegebenenfalls durch ein C₁- bis C₄-Alkylradikal, durch ein C₁- bis C₄-Hydroxyalkylradikal und/oder durch eine Verbrückung Y substituiert ist, oder das gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder C₁- bis C₈-Alkoxyradikale substituiert sein kann,
- G⁵ und G⁶ stehen unabhängig voneinander für ein Wasserstoff- oder Halogenatom, ein C₁- bis C₄-Alkylradikal, ein C₁- bis C₄-Monohydroxyalkylradikal, ein C₂- bis C₄-Polyhydroxyalkylradikal, ein C₁- bis C₄-Aminoalkylradikal oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder ein C₁- bis C₄-Alkylradikal,
mit der Maßgabe, daß die Verbindungen der Formel (7) nur eine Verbrückung Y pro Molekül enthalten.

Die in Formel (7) verwendeten Substituenten sind erfindungsgemäß analog zu den obigen Ausführungen definiert.

Bevorzugte zweikernige Entwicklerkomponenten der Formel (7) sind insbesondere: N,N'bis-(β-Hydroxyethyl)-N,N'-bis-(4'-Aminophenyl)-1,3-diamino-propanol, N,N'-bis-(β-Hydroxyethyl)-N,N'-bis-(4'-aminophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N'-bis-(Ethyl)-N,N'-bis(4'-amino,3'-methylphenyl)-ethylendiamin, 1,8-bis-(2,5-Diaminophenoxy)-3,5-dioxaoctan, Bis-(2-hydroxy-5-aminophenyl)-methan, 1,4-Bis-(4-aminophenyl)-diaza-cycloheptan und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (7) sind N,N'-bis-(β-Hydroxyethyl)-N,N'-bis-(4'-aminophenyl)-1,3-diamino-propanol, Bis-(2-hydroxy-5-aminophenyl)-methan, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (8) 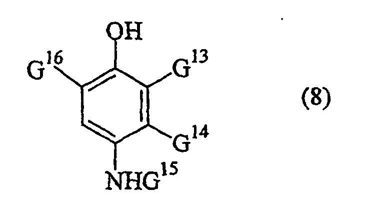 wobei:
- G¹³ steht für ein Wasserstoffatom, ein Halogenatom, ein C₁- bis C₄-Alkylradikal, ein C₁- bis C₄-Monohydroxyalkylradikal, ein (C₁- bis C₄)-Alkoxy-(C₁- bis C₄)-alkylradikal, ein C₁- bis C₄-Aminoalkylradikal, ein Hydroxy-(C₁- bis C₄)-alkylaminoradikal, ein C₁- bis C₄-Hydroxyalkoxyradikal, ein C₁- bis C₄-Hydroxyalkyl-(C₁-bis C₄)-aminoalkylradikal oder ein (Di-C₁- bis C₄-Alkylamino)-(C₁- bis C₄)-alkylradikal, und
- G¹⁴ steht für ein Wasserstoff- oder Halogenatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein C₁- bis C₄-Aminoalkylradikal oder ein C₁- bis C₄-Cyanoalkylradikal,
- G¹⁵ steht für Wasserstoff, ein C₁- bis C₄-Alkylradikal, ein C₁- bis C₄-Monohydroxyalkylradikal, ein C₂- bis C₄-Polyhydroxyalkylradikal, ein Phenylradikal oder ein Benzylradikal, und
- G¹⁶ steht für Wasserstoff oder ein Halogenatom.

Die in Formel (8) verwendeten Substituenten sind erfindungsgemäß analog zu den obigen Ausführungen definiert.

Bevorzugte p-Aminophenole der Formel (8) sind insbesondere p-Aminophenol, N-Methyl-p-Aminophenol, 4-Amino-3-methyl-phenol, 4-Amino-3-fluorphenol, 2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (8) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazolpyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamine-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (9) und dessen tautomeren Formen, sofern ein tautomerisches Gleichgewicht besteht: 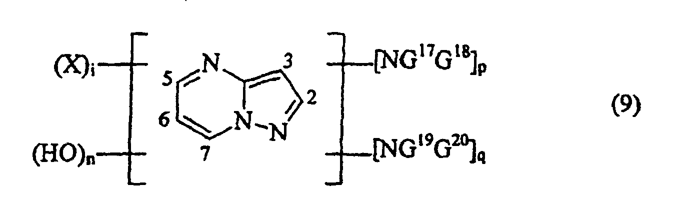 wobei:
- G¹⁷, G¹⁸, G¹⁹ und G²⁰ unabhängig voneinander stehen für ein Wasserstoffatom, ein C₁bis C₄-Alkylradikal, ein Aryl-Radikal, ein C₁- bis C₄-Hydroxyalkylradikal, ein C₂- bis C₄-Polyhydroxyalkylradikal ein (C₁- bis C₄)-Alkoxy-(C₁- bis C₄)-alkylradikal, ein C₁- bis C₄-Aminoalkylradikal, das gegebenenfalls durch ein Acetyl-Ureid- oder Sulfonyl-Radikal geschützt sein kann, ein (C₁- bis C₄)-Alkylamino-(C₁- bis C₄)-alkylradikal, ein Di-[(C₁- bis C₄)-alkyl]-(C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄)-[Hydroxyalkyl]-(C₁- bis C₄)-aminoalkylradikal,
- die X-Radikale stehen unabhängig voneinander für ein Wasserstoffatom, ein C₁- bis C₄-Alkylradikal, ein Aryl-Radikal, ein C₁- bis C₄-Hydroxyalkyladikal, ein C₂- bis C₄-Polyhydroxyalkylradikal, ein C₁- bis C₄-Aminoalkylradikal, ein (C₁- bis C₄)-Alkylamino-(C₁bis C₄)-alkylradikal, ein Di-[( C₁- bis C₄)alkyl]- (C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄-hydroxyalkyl)-aminoalkylradikal, ein Aminoradikal, ein C₁- bis C₄-Alkyl- oder Di-(C₁- bis C₄hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (9) verwendeten Substituenten sind erfindungsgemäß analog zu den obigen Ausführungen definiert.

Wenn das Pyrazol-[1,5-a]-pyrimidin der obenstehenden Formel (9) eine Hydroxygruppe an einer der Positionen 2, 5 oder 7 des Ringsystems enthält, besteht ein tautomeres Gleichgewicht, das zum Beispiel im folgenden Schema dargestellt wird:

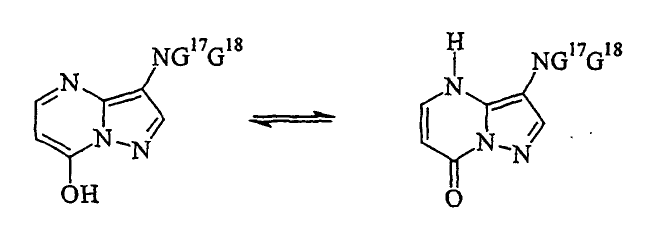

Unter den Pyrazol-[1,5-a]-pyrimidinen der obenstehenden Formel (9) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (9) können wie in der Literatur beschrieben durch Zyklisierung ausgehend von einem Aminopyrazol oder von Hydrazin hergestellt werden.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind:
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die Oxidationsfarbstoffvorprodukte sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Es kann erfindungsgemäß bevorzugt sein, die reaktiven Verbindungen getrennt von den Derivaten des Benzo[b]furan-3-ons und/oder des Benzo[b]thiophen-3-ons sowie den Oxidationsfarbstoffen zu lagern, so daß eine vorzeitige Reaktion dieser Komponenten ausgeschlossen werden kann. So können diese Verbindungen in getrennten wäßrigen Medien, wie beispielsweise Emulsionen gelagert werden, die erst unmittelbar vor der Anwendung auf den Fasern vereinigt werden.

Ferner kann auch eine oder mehrere der Komponenten in einem oder mehreren wäßrigen Medien und die andere(n) Komponente(n) als Pulver konfektioniert sein. Die pulverförmigen Komponenten werden im Rahmen dieser Ausführungsform unmittelbar vor der Anwendung zu dem wäßrigen Medium gegeben. Im Rahmen dieser Konfektionierung kann es erfindungsgemäß bevorzugt sein, die Komponente enthaltend die Verbindungen der Formel (1) als Pulver zu formulieren.

Weiterhin kann eine vorzeitige Reaktion auch vermieden werden, wenn die Komponenten gemeinsam als Pulverhaarfarbe formuliert werden. Die Komponenten werden dann erst unmittelbar vor der Anwendung auf den Fasern durch Zugabe einer wäßrigen Komponente aktiviert.

Abschließend sei auch noch die Möglichkeit erwähnt, die Komponenten getrennt in einem inerten Medium, wie beispielsweise einem inerten Öl, zu dispergieren.

Es kann erfindungsgemäß bevorzugt sein, die getrennt formulierten Komponenten - gegebenenfalls nach Wasserzugabe - nacheinander anzuwenden, wobei eventuell nach Anwendung der ersten Komponente eine Zwischenspülung durchgeführt werden kann.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (10a), 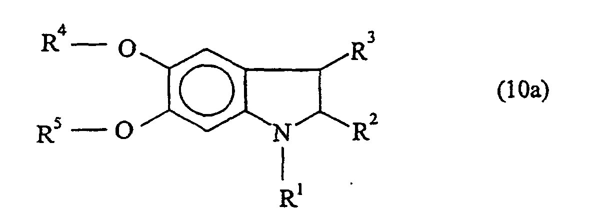 in der unabhängig voneinander
R¹ steht für Wasserstoff, eine C₁-C₄-Alkylgruppe oder eine C₁-C₄-Hydroxy-alkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (10b), 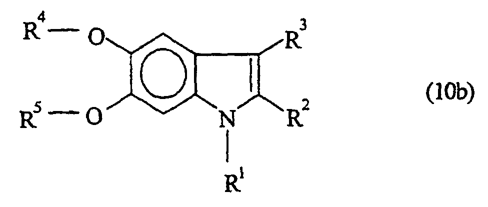 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch
als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

Neben den Farbstoffvorprodukten können die erfindungsgemäßen Mittel zur weiteren Nuancierung direktziehende Farbstoffe enthalten. Diese sind üblicherweise ausgewählt aus Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel enthalten.

Die erfindungsgemäßen Mittel enthalten die erfindungswesentlichen Komponenten bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, diese Verbindungen in eine pulverförmige oder auch Tabletten-förmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase, Ascorbat-Oxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Ein Vorteil des erfindungsgemäßen Färbesystems ist seine Stabilität gegenüber den in der oxidativen Haarfärbung üblicherweise eingesetzten Oxidationsmitteln. So kann das erfindungsgemäße Haarfärbesystem nicht nur als alleinige Färbekomponenten sondern beispielsweise auch zur Nuancierung oder zur Verbesserung der Echtheitseigenschaften oxidativer Färbemittel eingesetzt werden. Im Rahmen dieser Ausführungsform enthält das Mittel bevorzugt ein Oxidationsmittel; Wasserstoffperoxid ist erfindungsgemäß besonders bevorzugt.

Falls das Färbemittel nicht eine Einkomponentenprodukt darstellt, wird es zweckmäßigerweise erst unmittelbar vor der Anwendung durch Mischung der Zubereitungen, enthaltend die Farbstoffvorprodukte, und gegebenenfalls der Zubereitung des Oxidationsmittels hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu.

Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die den erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die im Rahmen des erfindungsgemäßen Verfahrens eingesetzten Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten. Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquatemium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und saure Aminosäuren sowie Basen und alkalische Aminosäuren,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der vorliegenden Erfindung ist ein Verfahren zum Färben keratinischer Fasern, bei dem ein erfindungsgemäßes Mittel auf die Fasern aufgetragen wird und nach einer Einwirkungszeit wieder abgespült wird.

Ein dritter Gegenstand der vorliegenden Erfidnung ist die Verwendung der erfindungsgemäßen Mittel zum Färben keratinischer Fasern.

Die folgenden Beispiele sollen den Gegenstand der vorliegenden Anmeldung verdeutlichen.

### Ausführungsbeispiele

### 1 Synthese der 3-Hydroxy-5-oxy-benzo[b]thiophene (Tautomere der 5-Oxybenzo[b]thiophen-3-one)

### 1.1 Synthese von 3,5-Dihydroxy-benzo[b]thiophen (I)

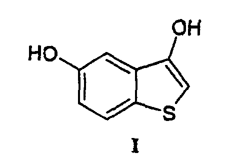

Diese Verbindung wurde gemäß A. Pawlik, *Dissertation*, Techn. Universität Braunschweig **1995**, 98-101 hergestellt.

### 1.2 Synthese des 5-Carboxymethoxy-3-hydroxy-benzo[b]thiophens

Dieses Molekül wurde nach Syntheseschema 1 hergestellt:

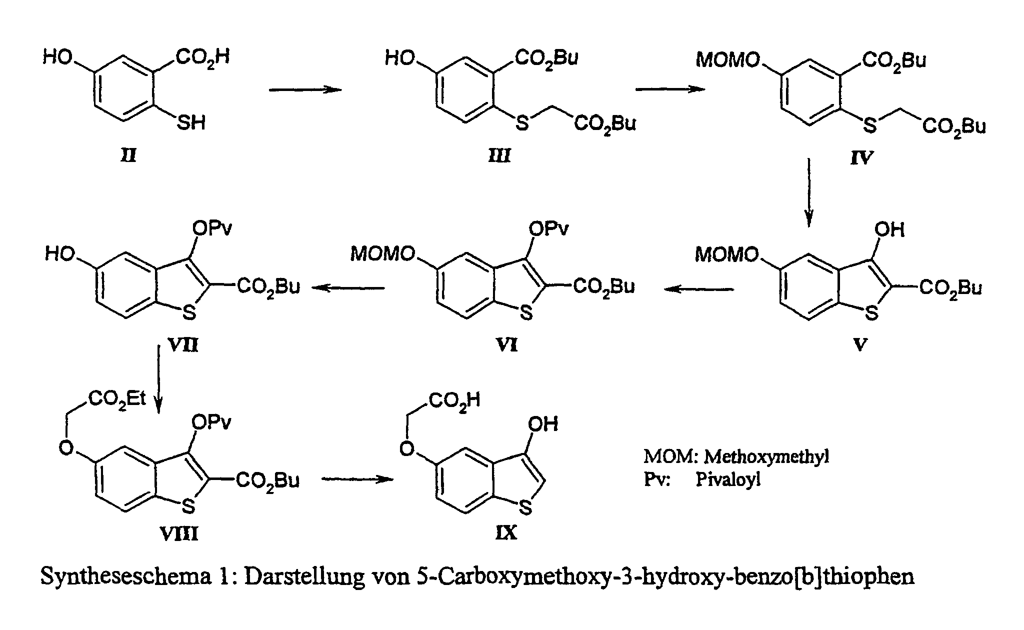

### 1.2.1 Synthese von 5-Hydroxy-2-mercapto-benzoesäure (II)

Diese Verbindung wurde gemäß A. Pawlik, *Dissertation*, Techn. Universität Braunschweig **1995**, 98-101 hergestellt.

### 1.2.2 Synthese von 5-Hydroxy-2-butoxycarbonylmethylsulfanyl-benzoesäurebutylester (III)

83.40 g (490 mmol) **II**, 196.00 g (4.90 mol) NaOH und 102.13 g (735 mmol) Bromessigsäure wurden in 1.5 L MeOH und 600 mL Wasser 32 h unter Rückfluß erhitzt. Anschließend wurde mit konzentrierter HCl pH I eingestellt, bevor die Lösemittel im Vakuum entfernt wurden. Der Rückstand wurde mit 1.5 L *n*-Butanol und 15 mL konzentrierter H₂SO₄ 12 h am Wasserabscheider erhitzt. Nach Entfernen des Lösemittels wurde der Rückstand mit 1 L Chloroform aufgenommen, einmal mit Wasser gewaschen und über Na₂SO₄ getrocknet Das Lösemittel wurde entfernt. Nach Lösen des Rückstandes in Ethylacetat erhielt man durch Ausfällen mit *n*-Pentan 101.80 g (299 mmol, 61 %) **III**.

### 1.2.3 Synthese von 5-Methoxymethyloxy- 2-butoxycarbonylmethylsulfanylbenzoesäurebutylester (IV)

Unter einer mit 3 A Molsieb gefüllten Extraktionshülse wurden 1.70 g (5 mmol) **III**, 2.21 mL (25 mmol) Dimethoxymethan und eine Spatelspitze *p*-Toluolsulfonsäure in 75 mL Dichlormethan 72 h unter Rückfluß erhitzt. Anschließende Säulenfiltration (50 g SiO₂, *n*-Hexan : Ethylacetat = 5 : 1) lieferte 1.61 g (4.2 mmol, 84 %) **IV**.

### 1.2.4 Synthese von 2-Butoxycarbonyl-3-hydroxy-5-methoxymethyloxy-benzo[b]thiophen (V)

In 80 mL DMF wurden 6.20 g (16 mmol) **IV** und 0.80 g (20 mmol, 60 %) NaH 5 h bei Raumtemperatur gerührt. Das Lösemittel wurde entfernt und der Rückstand mit 250 mL CHCl₃ aufgenommen, dreimal mit Wasser gewaschen und über Na₂SO₄ getrocknet Nach Umkristallisation aus *n*-Hexan erhielt man 4.22 g (13.6 mmol, 85 %) **V**.

### 1.2.5 Synthese von 2-Butoxycarbonyl-5-methoxymethyloxy-3-pivaloyloxybenzo[b]thiophen (VI)

6.21 g (20 mmol) **V** und 3.70 mL (30 mmol) Pivaloylchlorid wurden 12 h bei Raumtemperatur in 150 mL Pyridin gerührt. Nach Entfernen des Lösemittels lieferte eine Flash-Chromatographie (200 g SiO₂, *n*-Hexan : Ethylacetat = 5 : 1 + 1% Essigsäure, mit CHCl₃ aufgetragen) 7.39 g (18.7 mmol, 94 %) **VI**.

### 1.2.6 Synthese von 2-Butoxycarbonyl-5-hydroxy-3-pivaloyloxy-benzo[b]thiophen (VII)

In 150 mL Essigsäure wurden 5.92 g (15 mmol) **VI** mit einigen Tropfen konzentrierter HCl versetzt und 10 h bei Raumtemperatur gerührt. Anschließend wurden zur Reaktionslösung 500 mL Wasser gegeben und mehrfach mit Et₂O extrahiert. Die vereinigten organischen Phasen wurden über Na₂SO₄ getrocknet und vom Lösemittel befreit. Eine Umkristallisation aus Diethylether : *n*-Hexan = 4 : 1 lieferte 4.21 g (12 mmol, 80 %) **VII**.

### 1.2.7 Synthese von 2-Butoxycarbonyl-5-ethoxycarbonylmethoxy-3-pivaloyloxybenzo[b]thiophen (VIII)

3.50 g (10 mmol) **VII**, 2.22 mL (20 mmol) Bromessigsäureethylester und 2.48 g (25 mmol) K₂CO₃ wurden in 250 mL Aceton 5 h unter Rückfluß erhitzt. Die Reaktionsmischung wurde filtriert. Das Filtrat wurde vom Lösemittel befreit. Nach Umkristallisation aus *n*-Hexan erhielt man 4.00 g (9.2 mmol, 92 %) **VIII**.

### 1.2.8 Synthese von 5-Carboxymethoxy-3-hydroxy-benzo[b]thiophens (IX)

In 300 mL entgastem Wasser wurden 1.96 g (4.5 mmol) **VIII** und 2.70 g (67.5 mmol) NaOH 15 h unter Rückfluß erhitzt. Anschließend wurde mit konzentrierter HCl pH 1 eingestellt und die wäßrige Phase dreimal mit Ethylacetat extrahiert. Die vereinigten organischen Phasen werden über Na₂SO₄ getrocknet. Eine Flash-Chromatographie (200 g SiO₂, Ethylacetat : Essigsäure = 100 : 1, Präadsorption: 10 g SiO₂/Ethanol) liefert 801 mg (3.6 mmol, 79 %) **IX**.

### 2 Synthese der 5,6-Dioxy-3-hydroxy-benzo[b]thiophen-Derivate (Tautomere der 5,6-Dioxybenzo[b]thiophen-3-on-Derivate)

Die 5,6-Dioxy-3-hydroxy-benzo[b]thiophen-Derivate wurden gemäß Syntheseschema 2 hergestellt.

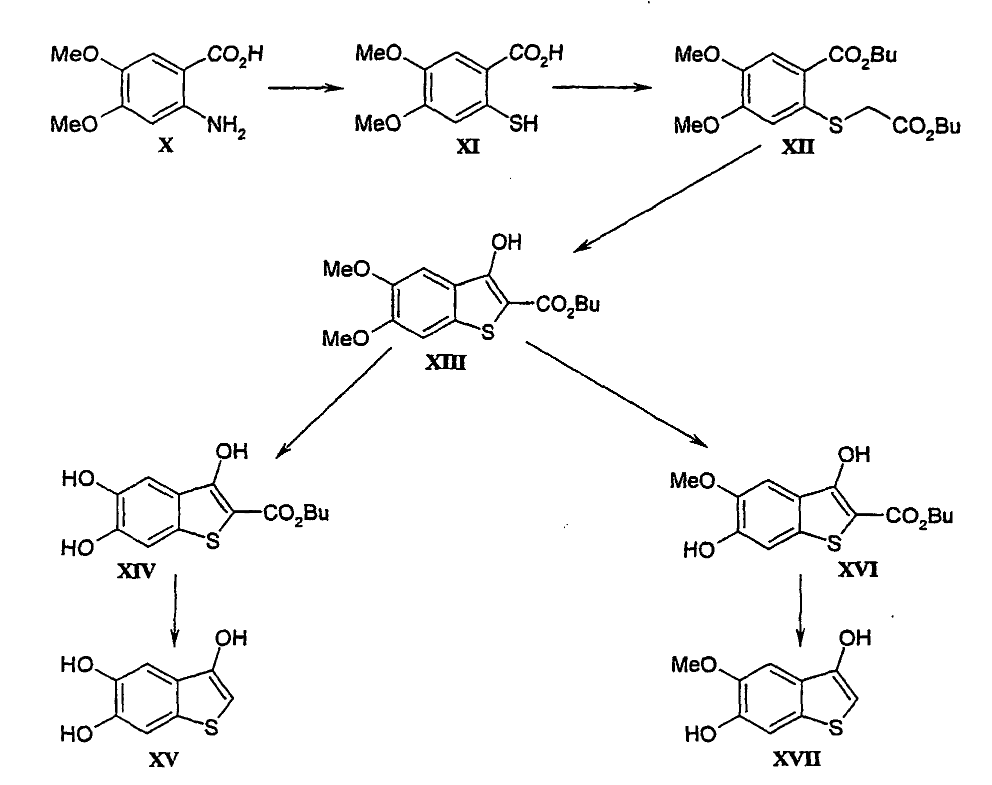

### 2.1 Synthese von 3,5,6-Trihydroxy-benzo[b]thiophen (XV)

### 2.1.1 Synthese von 4,5-Dimethoxy-2-mercapto-benzoesäure (XI)

Diese Verbindung wurde in Anlehnung an J. Szabò et al., *Acta Chim. Acad*. *Sci. Hung*. **1958**, *17*, 201-209 hergestellt.

### 2.1.2 Synthese von 4, 5-Dimethoxy-2-butoxycarbonylmethylsulfanylbenzoesäurebutylester (XII)

In einer Lösung aus 50 mL MeOH und 25 mL Wasser wurden 2.36 g (11 mmol) **XI**, 2.29 g (17 mmol) Bromessigsäure und 4.40 g (110 mmol) NaOH 24 h unter Rückfluß gerührt. Nach Ansäuern mit konzentrierter HCl auf pH 1 wurden die Lösemittel im Vakuum entfernt und der Rückstand mit 50 mL *n*-Butanol und 2 mL konzentrierter H₂SO₄ weitere 60 h am Wasserabscheider erhitzt.

Die Reaktionsmischung wurde vom Lösemittel befreit und der Rückstand mit 100 mL CHCl₃ aufgenommen. Nach Waschen der organischen Phase mit Wasser und Trocknen über Na₂SO₄ lieferte eine Flash-Chromatographie (100 g SiO₂, *n*-Hexan : Ethylacetat = 10: 1 + 1 % Essigsäure) 3.80 g (9.9 mmol, 90 %) **XII**.

### 2.1.3 Synthese von 2-Butoxycarbonyl-5,6-dimethoxy-3-hydroxy-benzo[b]thiophen (XIII)

19.22 g (50 mmol) **XII** wurden mit 2.75 g (63 mmol, 60 %) NaH in 250 mL DMF 3.5 h bei Raumtemperatur gerührt. Anschließend hydrolysierte man mit 50 mL gesättigter NH₄Cl-Lösung und entfernte die Lösemittel. Der Rückstand wurde in 500 mL CHCl₃ aufgenommen, filtriert, mit Wasser gewaschen und über Na₂SO₄ getrocknet. Nach Flash-Chromatographie (250 g SiO₂, *n*-Hexan : Ethylacetat = 5 : 1 + 1 % Essigsäure) erhielt man 14.70 g (47 mmol, 95 %) **XIII**.

### 2.1.4 Synthese von 2-Butoxycarbonyl-3,5,6-trihydroxy-benzo[b]thiophen (XIV)

Bei 0 °C wurden 2.79 g (9.0 mmol) **XIII** in 150 mL CH₂Cl₂ mit 2.64 mL (27 mmol) BBr₃ 2 h gerührt. Nach Hydrolyse mit 75 mL Wasser wurde mit Ethylacetat extrahiert. Die vereinigten organischen Phasen wurden über Na₂SO₄ getrocknet und man erhielt nach Flash-Chromatographie (200 g SiO₂, *n*-Hexan : Ethylacetat = 2 : 1 + 1 % Essigsäure) 2.48 g (8.8 mmol, 98 %) **XIV**.

### 2.1.5 Synthese von 3,5,6-Trihydroxy-benzo[b]thiophen (XV)

In 300 mL entgastem Wasser wurden 2.82 g (10 mmol) **XIV** und 6.00 g (150 mmol) NaOH 3.5 h unter Rückfluß erhitzt. Anschließend säuerte man die noch heiße Lösung mit konzentrierter HCl auf pH 1 an. Das beim Abkühlen im Eisbad ausfallende **XV** wurde abgetrennt und mit wenig Diethylether gewaschen. Man erhielt 1.57 g (8.6 mmol, 86 %) **XV**.

### 2.2 Synthese von 5-Methoxy-3,6-dihydroxy-benzo[b]thiophen (XVII)

### 2.2.1 Synthese von 2-Butoxycarbonyl-3,6-dihydroxy-5-methoxy-benzo[b]thiophen (XVI)

4.45 mL (60 mmol) Ethanthiol wurden in 100 mL DMF mit 2.62 g (60 mmol, 60 %) NaH 15 min bei Raumtemperatur gerührt. Zu dieser Lösung wurden 9.31 g (30 mmol) **XIII** gegeben und 4 h unter Rückfluß erhitzt. Nach Hydrolyse mit 25 mL gesättigter NH₄Cl-Lsg wurden die Lösemittel im Vakuum entfernt, der Rückstand mit 500 mL Ethylacetat aufgenommen und mit Wasser gewaschen. Nach Trocknen über Na₂SO₄ lieferte eine Flash-Chromatographie (250 g SiO₂, *n*-Hexan : Ethylacetat = 2 : 1 + 1 % Essigsäure, Präadsorption: 25 g SiO₂/CHCl₃) 6.89 g (23 mmol, 78 %) **XVI**.

### 2. 2.2 Synthese von 3,6-Dihydroxy-5-methoxy-benzo[b]thiophen (XVII)

In 100 mL entgastem Wasser wurden 2.96 g (10 mmol) **XVI** und 6.00 g NaOH 3.5 h unter Rückfluß erhitzt. Anschließend wurde die noch heiße Lösung mit konzentrierter HCl auf pH 1 angesäuert. Das beim Abkühlen im Eisbad ausfallende **XVII** wurde abgetrennt und mit wenig *n*-Hexan gewaschen. Man erhielt 1.23 g (6.3 mmol, 63 % mmol) **XVII**.
Durch Extraktion des wäßrigen Filtrats mit Ethylacetat und anschließender Flash-Chromatographie (50 g SiO₂, *n*-Hexan : Ethylacetat = 2 : 1 + 1 % Essigsäure, Präadsorption: 2 g SiO₂/Ethylacetat) ließ sich die Ausbeute an **XVII** auf insgesamt 1.73 g (8.8 mmol, 88 %) steigern.

### 3 Synthese der 5-Oxy-benzo[b]furan-3-on-Derivate

Die 5-Oxy-benzo[*b*]furan-3-on-Derivate wurden gemäß Syntheseschema 3 hergestellt.

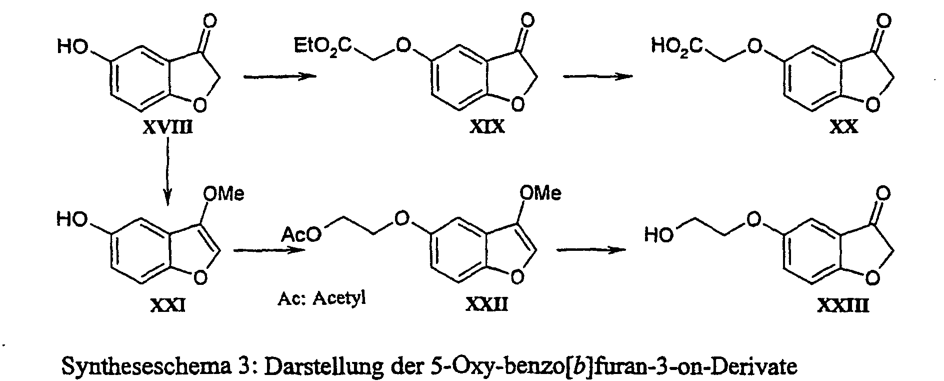

### 3.1 Synthese von 5-Hydroxy-benzo[b]furan-3-on (XVIII)

Das 5-Hydroxy-benzo[*b*]furan-3-on (**XVIII**) wurde gemäß einer Vorschrift von M. C. Kloetzel et al., *J*. *Org. Chem.* **1955,** *20*, 38-49 synthetisiert.

### 3.2 Synthese von 5-Carboxymethoxy-benzo[b]furan-3-on (XX)

### 3.2.1 Synthese von 5-Ethoxycarbonylmethoxy-benzo[b]furan-3-on (XIX)

In 150 mL Tetrahydrofuran wurden 3.00 g (20 mmol) **XVIII**, 3.96 g (40 mmol) K₂CO₃ und 5.54 mL (50 mmol) Bromessigsäureethylester 72 h unter Rückfluß erhitzt. Anschließend wurde filtriert und das Lösemittel entfernt. Eine Flash-Chromatographie (200 g SiO₂, *n*-Hexan : Ethylacetat = 2 : 1) lieferte 4.63 g (19.6 mmol, 98 %) **XIX**.

### 3.2.2 Synthese von 5-Carboxymethoxy-benzo[b]furan-3-on (XX)

In einer Mischung aus 200 mL Tetrahydrofuran, 100 mL Wasser und 10 mL konzentrierter H₂SO₄ wurden 4.72 g (20 mmol) **XIX** 15 h unter Rückfluß erhitzt. Die Reaktionslösung wurde mit Ethylacetat und gesättigter NaCl-Lösung versetzt. Die Phasen wurden getrennt und die wäßrige dreimal mit Ethylacetat extrahiert. Die vereinigten organischen Phasen wurden über Na₂SO₄ getrocknet und vom Lösemittel befreit. Durch Flash-Chromatographie (200 g SiO₂, *n*-Hexan : Ethylacetat = 1 : 2 + 1 % Essigsäure, mit Ethanol aufgetragen) erhielt man 2.78 g (13 mmol, 67 %) **XX**.

### 3.3 Synthese von 5-(2'-Hydroxyethoxy-benzo[b]furan-3-on (XXIII)

### 3.3.1 Synthese von 5-Hydroxy-3-methoxy-benzo[b]furan (XXI)

900 mg (6.0 mmol) **XVIII**, 788 µl (7.2 mmol) Orthoameisensäuretrimethylester und 57 mg (0.3 mmol) *p*-Toluolsulfonsäure Monohydrat wurden in 70 mL Methanol 12 h unter Rückfluß erhitzt. Die Reaktion wurde durch Zugabe gesättigter NaHCO₃-Lösung gestoppt und die Lösemittel entfernt. Die anschließende Flash-Chromatographie (100 g SiO₂, *n*-Hexan : Ethylacetat = 2 : 1, mit Ethanol aufgetragen) lieferte 717 mg (4.4 mmol, 73 %) **XXI**.

### 3.3.2 Synthese von 3-Methoxy-5-(2'-Acetoxyethoxy)-benzo[b]furan (XXII)

Bei 0 °C wurden 164 mg (1.0 mmol) **XXI** und 48 mg (1.2 mmol, 60 %) NaH in 20 mL DMF 30 min gerührt. Zu dieser Suspension gab man 227 µl (2.0 mmol, 97 %) 2-Bromethylacetat und rührte 72 h bei Raumtemperatur. Durch Zugabe von 3 mL gesättigter NH₄Cl-Lösung quenchte man die Reaktion und entfernte die Lösemittel im Vakuum. Der Rückstand wurde mit Ethylacetat und Wasser aufgenommen, die wäßrige Phase noch zweimal mit Ethylacetat extrahiert und die vereinigten organischen Phasen über Na₂SO₄ getrocknet. Nach Säulenfiltration (40 g SiO₂, *n*-Hexan : Ethylacetat = 2 : 1, mit Ethylacetat aufgetragen) erhielt man 220 mg (0.88 mmol, 88 %) **XXII**.

### 3.3.3 Synthese von 5-(2'-Hydroxyethoxy)-benzo[b]furan-3-on (XXIII)

In einer Mischung aus 40 mL Tetrahydrofuran, 20 mL Wasser und 0.5 mL konz. H₂SO₄ wurden 751 mg (7.0 mmol) **XXII** 12 h unter Rückfluß gerührt. Die auf Raumtemperatur abgekühlte Reaktionsmischung wurde mit Ethylacetat und gesättigter NaCl-Lösung versetzt. Nach Trennen der Phasen wurde die wäßrige Phase noch zweimal mit Ethylacetat extrahiert und die vereinigten organischen Phasen über Na₂SO₄ getrocknet. Nach Säulenfiltration (50 g SiO₂, *n*-Hexan : Ethylacetat = 1 : 1 + 1 % Essigsäure, mit Ethylacetat aufgetragen) erhielt man 448 mg (2.3 mmol, 77 %) **XXIII**.

### 4 Anwendungsbeispiele

Die Mengenangaben verstehen sich, sofern nichts anderes angegeben ist, in Gewichtsprozent.

Es wurden die folgenden Abkürzungen verwendet:

| | | |
|---|---|---|
| 1 | 5-Hydroxy-benzo[b]thiophen-3-on | (I) |
| 2 | 5-Carboxymethoxy-benzo[b]thiophen-3-on | (IX) |
| 3 | 5,6-Dihydroxy-benzo[b]thiophen-3-on | (XV) |
| 4 | 6-Hydroxy-5-methoxy-benzo[b]thiophen-3-on | (XVII) |
| 5 | 5-Carboxymethoxy-benzo[b]furan-3-on | (XX) |
| 6 | 5-Hydroxy-benzo[b]furan-3-on | (XVIII) |
| 7 | 5-(2'-Hydroxyethoxy)-benzo[b]furan-3-on | (XXIII) |
| A | 4-N,N-Dimethylamino-benzaldehyd | |
| B | 4-N,N-Dimethylamino-zimtaldehyd | |
| C | Vanillin | |
| D | 4-N,N-Dimethylamino-1-naphthaldehyd | |
| E | Isatin | |

### 4.1 Versuchsreihe A

Zunächst wurden jeweils 0,1g der Farbstoffe 1 bis 7 mit der entsprechenden äquimolaren Menge an Carbonylverbindung A bis E, 0,1g Natrosol® HR250¹ und 0,05g Ammoniumsulfat vermischt, mit 9g Wasser versetzt und der pH-Wert mit einer 25%igen Ammoniaklösung auf einen Wert von 10 eingestellt Anschließend wurde diese Quellung mit Wasser auf 10g aufgefüllt.
¹ Hydroxyethylcellulose (INCI-Bezeichnung: Hydroxyethylcellulose) (Hercules)

Unmittelbar vor der Anwendung wurden diese Lösungen für die Reaktion ohne Wasserstoffperoxid im Verhältnis 1:1 mit Wasser vermischt. Im Falle der Reaktion mit Wasserstoffperoxid wurden diese Lösungen entsprechend im Verhältnis 1:1 mit einer 3%igen wäßrigen Wasserstoffperoxidlösung (Marktprodukt Poly Color Tönungswäsche) vermischt

In 10g dieser Anwendungszubereitung wurde je eine Haarsträhne (Alkinco Virgin-White, 0,5g) für 30min bei Raumtemperatur getaucht, anschließend mit Wasser gespült und getrocknet.

Die färberischen Ergebnisse sind in Tabelle I zusammengefaßt.

**Tabelle I:**

| **Verbdg. Nr.** | **Carbonylverbindung** | **Farbnuance** | |
|---|---|---|---|
| | | **Ohne H**_{**2**}**O**_{**2**} | **Mit H**_{**2**}**O**_{**2**} |
| 1 | A | hochrot | rot |
| 1 | B | violettbraun | violettbraun |
| 1 | C | goldgelb | graugrün |
| 1 | D | braunorange | grauorange |
| 1 | E | rotorange | orange |
| 2 | A | orangerot | rotorange |
| 2 | B | hellbraun | hellbraun |
| 2 | C | hellorange | gelbgrau |
| 2 | D | graurot | blaßrot |
| 3 | A | dunkelorange | dunkelorange |
| 3 | B | rotorange | rotorange |
| 3 | C | orangegelb | olivgelb |
| 3 | D | orange | graugelb |
| 4 | A | orange | orange |
| 4 | B | grauorange | grauorange |
| 4 | C | gelb | oliv |
| 4 | D | hellorange | oliv |
| 5 | A | hellorange | hellorange |
| 5 | B | orange | orange |
| 5 | C | hellgelb | grauorange |
| 5 | D | hellorange | blaßorange |
| 6 | A | rotorange | orange |
| 6 | B | rot | orangerot |
| 6 | C | orangegelb | gelb |
| 6 | D | orange | hellorange |
| 7 | A | rotorange | orange |
| 7 | B | rot | orangerot |
| 7 | C | gelb | pastellgelb |
| 7 | D | orange | hellorange |

### 4.2 Versuchsreihe B

Zunächst wurden jeweils 0,1g der Farbstoffe 1, 4, 6 und 7 und jeweils äquimolare Mengen an Carbonylverbindung A bis D entsprechend den Angaben in Tabelle II mit 0,1g Natrosol® HR250¹ und 0,05g Ammoniumsulfat vermischt, mit 9g Wasser versetzt und der pH-Wert mit einer 25%igen Ammoniaklösung auf einen Wert von 10 eingestellt. Anschließend wurde diese Quellung mit Wasser auf 10g aufgefüllt.

5g dieser Färbemischung wurden mit 5g einer Zubereitung, enthaltend die übrigen jeweils in Tabelle II angegebenen Rohstoffe, vermischt. Unmittelbar vor der Anwendung wurden diese Lösungen im Verhältnis 1:1 mit einer 3%igen wäßrigen Wasserstoffperoxidlösung (Marktprodukt Poly Color Tönungswäsche) vermischt.

Diese Anwendungszubereitung wurde im Gewichtsverhältnis 4:1 auf je eine Haarsträhne (Alkinco Virgin-White) gegeben. Die Einwirkungszeit betrug 30min; anschließend wurde mit Wasser gespült und getrocknet.

Die färberischen Ergebnisse sind in Tabelle III zusammengefaßt.

**Tabelle II:**

| **Rohstoff** | **Rezeptur-Nr. / Menge Gew.-%** | | | |
|---|---|---|---|---|
| | **1** | **2** | **3** | **4** |
| Fettalkoholgemisch C₁₂-C₁₈ | 5,75 | 5,75 | 5,75 | 5,75 |
| Eumulgin® B2² | 0,5 | 0,5 | 0,5 | 0,5 |
| Texapon® NSO³ | 6,25 | 6,25 | 6,25 | 6,25 |
| Polymer® JR⁴ | 0,4 | 0,4 | 0,4 | 0,4 |
| Natriumsulfit | 0,15 | 0,15 | 0,15 | 0,15 |
| Ammoniumsulfat | 0,25 | 0,25 | 0,25 | 0,25 |
| Natrosol® 250 HR | 0,5 | 0,5 | 0,5 | 0,5 |
| 2,4,5,6-Tetraaminopyrimidin-sulfat | - | -- | 0,76 | -- |
| p-Toluylendiamin-sulfat | 0,164 | -- | 0,092 | -- |
| p-Phenylendiamin-dihydrochlorid | - | 0,092 | -- | 0,08 |
| 3-Methyl-4-aminophenol | 0,015 | 0,017 | 0,012 | 0,01 |
| 2-Aminomethyl-4-aminophenol-dihydrochlorid | - | -- | 0,021 | 0,01 |
| Resorcin | 0,061 | 0,038 | 0,36 | -- |
| 2-Methylresorcin | 0,014 | -- | 0,22 | -- |
| 3-Aminophenol | 0,008 | 0,0034 | 0,004 | -- |
| 2,7-Dihydroxynaphthalin | 0,004 | -- | 0,28 | -- |
| 2-Methylamino-3-amino-6-methoxypyridin | 0,007 | -- | - | -- |
| 2-Amino-3-hydroxypyridin | 0,001 | -- | -- | 0,026 |
| 1,3-Bis-(2',4'-diaminophenoxy)-propan-tetrahydrochlorid | - | 0,0005 | -- | -- |
| 5-Amino-2-methylphenol | -- | -- | -- | 0,030 |
| A | 0,52 | -- | -- | -- |
| B | -- | 0,48 | -- | -- |
| C | -- | -- | 0,55 | -- |
| D | - | -- | - | 0,55 |
| 1 | 0,5 | -- | - | -- |
| 4 | -- | 0,5 | -- | -- |
| 6 | - | -- | 0,5 | -- |
| 7 | -- | -- | - | 0,5 |
| Wasser | ad 100 | ad 100 | ad 100 | ad 100 |

| | | | | |
|---|---|---|---|---|
| ² ethoxylierter Cetylstearylalkohol mit ca. 20-EO-Einheiten, (INCI-Bezeichnung: Ceteareth-20) (Cognis) | | | | |
| ³ Laurylethersulfat-Natrium-Salz, (INCI-Bezeichnung: Sodium Laureth Sulfate, ca. 28% Aktivsubstanz) (Cognis) | | | | |
| ⁴ quaternierte Hydroxyethylcellulose, (INCI-Bezeichnung: Polyquaternium-10) (Amerchol) | | | | |

**Tabelle III:**

| Rezeptur-Nr. | Färbung |
|---|---|
| 1 | Braunorange |
| 2 | Hellbraun |
| 3 | Orangerot |
| 4 | Hellbraun |

### 4.3 Versuchsreihe C

### Versuchsdurchführung:

Es wurden 15mMol 3,5-Dihydroxy-benzo[b]thiophen (Tabelle VI) beziehungsweise 15mMol 5-Hydroxybenzo[b]furan-3-on (Tabelle V) sowie 15mMol der in Tabelle IV beziehungsweise Tabelle V genannten Carbonylverbindungen jeweils separat in 100g einer 1Gew.-%igen Natrosol® 250 HR-Quellung gelöst, die zusätzlich 0,5Gew.-% Ammoniumsulfat enthielt

Für die Ausfärbungen ohne Wasserstoffperoxid wurden unmittelbar vor dem Ausfärben 2,5g der jeweiligen Quellungen enthaltend die beiden Komponenten im miteinander vermischt.

Bei den Ausfärbungen mit Wasserstoffperoxid wurden 1,25g der jeweiligen Quellungen der beiden Komponenten mit 2,5g einer 3%igen Entwicklerzubereitung (Poly Color Tönungswäsche) versetzt.

Anschließend wurde in beiden Fällen der pH-Wert gemäß den Angaben in Tabelle IV beziehungsweise Tabelle V mit Weinsäure bzw. Ammoniaklösung (25%ig) eingestellt.

Die Ausfärbungen erfolgten auf Wollläppchen (Abmessungen 4 x 3 cm) des Codes ISO105/F mit je 5g der anwendungsfertigen Formulierungen bei 32°C mit einer Einwirkdauer von 30 Minuten. Nach dem Abspülen und Trocknen wurden folgende Ergebnisse erhalten:
(Farbcodes nach dem Taschenlexikon der Farben, Auflage 1975 Musterschmidt-Verlag, Zürich, Göttingen)

**Tabelle IV:**

| Farbergebnisse mit 3,5-Dihydroxy-benzo[b]thiophen (I) | | | |
|---|---|---|---|
| Carbonylverbindungen | Farbresultat | | |
| | Ohne H₂O₂ | | mit H₂O₂ |
| | pH 10 | pH 6 | pH 10 |
| Anthracen-9-aldehyd | Graugelb | graugelb | gelbgrau |
| 4-Hydroxy-3-methoxyzimtaldehyd | Tomantenrot | englischrot | olivgrau |
| Terephthalaldehyd | Graurot | graurot | graurot |
| Isophthalaldehyd | Hellorange | möhrenrot | blaßgelb |
| 1-Methylindol-3-aldehyd | Kupfer | grauorange | grauorange |
| 4-Nitrobenzaldehyd | Braunrot | braunrot | blaßorange |
| Indol-3-aldehyd | Grauorange | hellorange | grauorange |
| 4-Formyl-1-methylchinolinium-p-toluolsulfonat | Graurubin | grauviolett | blaugrün |
| 2-Formyl-1-methylchinolinium-p-touolsulfonat | Violettgrau | purpurgrau | nilgrün |

**Tabelle V:**

| Farbergebnisse mit 5-Hydroxybenzo[b]furan-3-on (XVIII) | | | |
|---|---|---|---|
| Carbonylverbindungen | Farbresultat | | |
| | Ohne H₂O₂ | | mit H₂O₂ |
| | pH 10 | pH 6 | pH 10 |
| Anthracen-9-aldehyd | Hellorange | blaßgelb | hellorange |
| 4-Hydroxy-3-methoxyzimtaldehyd | Braungelb | braunorange | ockergelb |
| Terephthalaldehyd | Sonnengelb | kadmiumgelb | ginstergelb |
| Isophthalaldehyd | Schwefelgelb | neapelgelb | sonnengelb |
| 1-Methylindol-3-aldehyd | Orange | gelborange | orangegelb |
| 4-Nitrobenzaldehyd | Hellgelb | hellgelb | korngelb |
| Indol-3-aldehyd | Hellorange | hellgelb | bernsteingelb |
| 4-Formyl-1-methylchinolinium-p-toluolsulfonat | Mattviolett | blaugrau | braungrau |
| 2-Formyl-1-methylchinolinium-p-touolsulfonat | Graublau | mattblau | graugrün |

## Patentansprüche

1. Mittel zur Färbung keratinischer Fasern, enthaltend in einem kosmetisch akzeptablen Medium als Farbstoffvorprodukte
(a) mindestens ein Derivat des Benzo[b]furan-3-ons und/oder mindestens ein Derivat des Benzo[b]thiophen-3-ons der Formel (1), 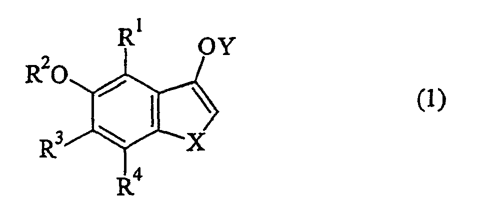 worin
X steht für Schwefel oder Sauerstoff,
Y steht für Wasserstoff, eine C₁₋₄-Alkylgruppe oder einen C₁₋₄-Acylrest und
die Reste R¹, R³ und R⁴ stehen unabhängig voneinander für
- Wasserstoff,
- eine C₁₋₄-Alkylgruppe, die gegebenenfalls mit
- einer oder mehreren Hydroxygruppe(n)
- einer gegebenenfalls substituierten Aminogruppe,
- einer gegebenenfalls mit einer C₁₋₄-Alkylgruppe veresterten Carboxygruppe oder
- einer Gruppe -SO₂R⁵, wobei R⁵ steht für eine Hydroxygruppe, eine gegebenenfalls substituierte Aminogruppe oder eine C₁₋₄-Alkylgruppe
substituiert sein kann,
- eine gegebenenfalls substituierte Aminogruppe,
- eine perfluorierte C₁₋₄-Alkylgruppe,
- eine Cyangruppe,
- eine C₂₋₅-Alkenylgruppe,
- eine Nitrogruppe,
- ein Halogenatom,
- eine gegebenenfalls mit einer C₁₋₄-Alkylgruppe substituierte Mercaptogruppe,
- eine Gruppe -SO₂R⁵ oder
- eine Gruppe -OR⁶,
- eine gegebenenfalls veresterte oder amidierte Carboxylgruppe und R² und gegebenenfalls R⁶ stehen unabhängig voneinander für
- Wasserstoff,
- eine C₁₋₄-Alkylgruppe, die gegebenenfalls mit
- einer oder mehreren Hydroxygruppe(n),
- einer gegebenenfalls substituierten Aminogruppe,
- einer gegebenenfalls mit einer C₁₋₄-Alkylgruppe veresterten Carboxygruppe oder
- einer Gruppe -SO₂R⁵
substituiert sein kann, oder
- eine perfluorierte C₁₋₄-Alkylgruppe,
und
(b) mindestens eine reaktive Verbindung ausgewählt aus
(b1) den reaktiven Carbonylverbindung, ausgewählt aus der Gruppe der aromatischen, heteroaromatischen oder ungesättigten Aldehyde oder Ketone, der Dialdehyde oder Diketone oder der Acetale, Halbaminale oder Iminderivate solcher reaktiver Carbonylverbindungen und/oder
(b2) den CH-aktiven Verbindungen mit den Formeln (2) oder (3), 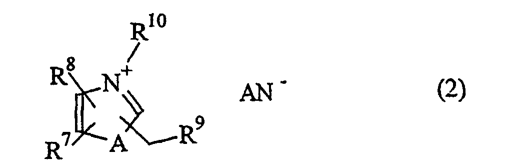 in der R¹⁰ steht für eine C₁₋₁₀-Alkylgruppe, eine C₂₋₄-Alkenylgruppe, eine C₂₋₄-Hydroxyalkylgruppe, eine C₂₋₄-Carboxyalkylgruppe, eine C₂₋₄-Sulfoalkylgruppe oder eine Aralkylgruppe,
R⁷ und R⁸ unabhängig voneinander ein Wasserstoffatom, eine C₁₋₄-Alkylgruppe, ein Halogenatom, eine Hydroxygruppe, eine C₁₋₄-Alkoxygruppe oder eine Nitrogruppe bedeuten oder zusammen einen ankondensierten aromatischen Ring bilden,
R⁹ steht für ein Wasserstoffatom, eine C₁₋₄-Alkylgruppe oder eine Arylgruppe,
A steht für ein Sauerstoff- oder ein Schwefelatom, die Gruppe -CH=CHoder >N-R¹¹, in der R¹¹ steht für eine C₁₋₄-Alkylgruppe, eine C₂₋₄-Carboxyalkylgruppe, eine C₂₋₄-Sulfoalkylgruppe, eine C₂₋₄-Sulfoxyalkylgruppe, eine C₂₋₄-Hydroxyalkylgruppe oder eine Aralkylgruppe, und
AN⁻ steht für ein Anion, das ausgewählt ist aus Halogenid, C₁₋₄-Alkylsulfat, C₁₋₄-Alkansulfonat, Arensulfonat, C₁₋₄-Perfluoralkansulfat, Tetrafluorborat, Perhalogenat, Sulfat, Hydrogensulfat oder Carboxylat, 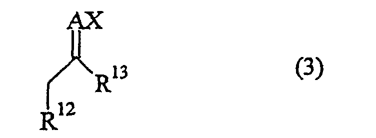 in der R¹² steht für eine C₁₋₄-Acylgruppe, eine C₁₋₄-Alkylsulfonylgruppe, eine C₁₋₄-Alkylsulfinylgruppe, eine C₁₋₄-Alkylaminogruppe, eine Di-C₁₋₄alkylaminogruppe, eine Vinylcarbonylgruppe, eine Methiniminogruppe, eine Nitrilgruppe, eine Ester- oder Carbonsäureamidgruppe, die gegebenenfalls durch eine C₁₋₄-Alkylgruppe, eine C₂₋₄-Hydroxyalkylgruppe oder eine Arylgruppe substituiert sein können, und
R¹³ steht für eine C₁₋₄-Acylgruppe, eine C₁₋₄-Alkoxygruppe, eine C₁₋₄-Alkylaminogruppe, eine C₁₋₄-Acylaminogruppe oder eine Di-C₁₋₄alkylaminogruppe,
wobei die Reste R¹² und R¹³ auch gemeinsam mit dem Restmolekül ein 5-, 6- oder 7-gliedriges Ringsystem aus der Reihe der Thiazolidin-2,5-dione, Thiazolidin-2-thion-5-one, Perhydropyrimidin-2,4,6-trione, Perhydropyrimidin-2-thion-4,6-dione, Cyclopentan-1,3-dione, Cyclohexan-1,3-dione, Indan-1,3-dione, 2-Pyrazolin-5-one, 1,2-Dihydro-6-hydroxy-2-hydroxypyridine oder deren Enolester bilden können, und
AX steht für Sauerstoff, Schwefel oder eine Dicyanmethylengruppe.

2. Mittel nach Anspruch 1, **dadurch gekennzeichnet, daß** Y für Wasserstoff steht

3. Mittel nach einem der Ansprüche 1 oder 2, **dadurch gekennzeichnet, daß** R¹ für Wasserstoff steht.

4. Mittel nach einem der Ansprüche 1 bis 3, **dadurch gekennzeichnet, daß** R⁴ für Wasserstoff steht

5. Mittel nach einem der Ansprüche 1 bis 4, **dadurch gekennzeichnet, daß** X für ein Sauerstoffatom steht

6. Mittel nach Anspruch 5, **dadurch gekennzeichnet, daß** das Benzo[b]furan-3-onderivat der Formel (1) ausgewählt ist aus 5-Carboxymethoxy-benzo[b]furan-3-on, 5-Hydroxy- benzo[b]furan-3-on oder 5-(2'-Hydroxyethoxy)-benzo[b]furan-3-on.

7. Mittel nach einem der Ansprüche 1 bis 4, **dadurch gekennzeichnet, daß** X steht für ein Schwefelatom.

8. Mittel nach Anspruch 7, **dadurch gekennzeichnet, daß** das Benzo[b]thiophen-3-onderivat der Formel (1) ausgewählt ist aus 5-Hydroxy-benzo[b]thiophen-3-on, 5-Carboxymethoxy-benzo[b]thiophen-3-on, 5,6-Dihydroxy-benzo[b]thiophen-3-on oder 6-Hydroxy-5-methoxy-benzo[b]thiophen-3-on.

9. Mittel nach einem der Ansprüche 1 bis 8, **dadurch gekennzeichnet, daß** es mindestens eine reaktive Carbonylverbindung vom Typ des Isatins und seinen Derivaten enthält.

10. Mittel nach einem der Ansprüche 1 bis 9, **dadurch gekennzeichnet, daß** es mindestens eine reaktive Carbonylverbindung vom Typ der aromatischen Aldehyde und deren Derivaten enthält.

11. Mittel nach einem der Ansprüche 9 oder 10, **dadurch gekennzeichnet, daß** die reaktive Carbonylverbindung ausgewählt ist aus 4-N,N-Dimethylamino-benzaldehyd, 4-N,N-Dimethylamino-zimtaldehyd, Vanillin, 4-N,N-Dimethylamino-1-naphthaldehyd und Isatin.

12. Mittel nach einem der Ansprüche 1 bis 11, **dadurch gekennzeichnet, daß** es weiterhin mindestens ein Oxidationsfarbstoffvorprodukt vom Entwickler- und/oder Kupplertyp enthält.

13. Mittel nach einem der Ansprüche 1 bis 12, **dadurch gekennzeichnet, daß** es weiterhin mindestens ein Farbstoffvorprodukt vom Indol- oder Indolintyp enthält.

14. Mittel nach einem der Ansprüche 1 bis 13, **dadurch gekennzeichnet, daß** es weiterhin mindestens einen direktziehenden Farbstoff enthält.

15. Mittel nach einem der Ansprüche 1 bis 14, **dadurch gekennzeichnet, daß** es ein Oxidationsmittel, vorzugsweise Wasserstoffperoxid, enthält.

16. Verwendung eines Mittels nach einem der Ansprüche 1 bis 15 zum Färben keratinischer Fasern.

17. Verfahren zur Färbung keratinischer Fasern, bei dem die Fasern mit einem Mittel nach einem der Ansprüche 1 bis 16 behandelt und anschließend gespült werden.

## Claims

1. A composition for coloring keratinous fibers which contains as dye precursors in a cosmetically acceptable medium
(a) at least one derivative of benzo[b]furan-3-one and/or at least one derivative of benzo[b]thiophen-3-one corresponding to formula (1): 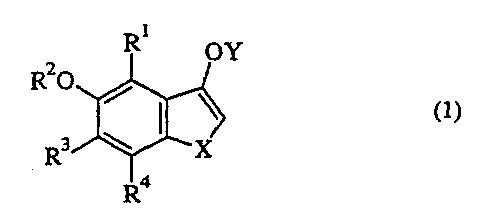 in which
X is sulfur or oxygen,
Y is hydrogen, a C₁₋₄ alkyl group or a C₁₋₄ acyl group,
the substituents R¹, R³ and R⁴ independently of one another represent
- hydrogen,
- a C₁₋₄ alkyl group which may optionally be substituted by
- one or more hydroxy group(s)
- an optionally substituted amino group,
- a carboxy group optionally esterified with a C₁₋₄ alkyl group or
- a group -SO₂R⁵, where R⁵ is a hydroxy group, an optionally substituted amino group or a C₁₋₄ alkyl group,
- an optionally substituted amino group,
- a perfluorinated C₁₋₄ alkyl group,
- a cyano group,
- a C₂₋₅ alkenyl group,
- a nitro group,
- a halogen atom,
- an optionally C₁₋₄-alkyl-substituted mercapto group,
- a group -SO₂R⁵ or
- a group -OR⁶,
- an optionally esterified or amidated carboxyl group and R² and optionally R⁶ independently of one another represent
- hydrogen,
- a C₁₋₄ alkyl group which may optionally be substituted by
- one or more hydroxy group(s),
- an optionally substituted amino group,
- a carboxy group optionally esterified with a C₁₋₄ alkyl group or
- a group -SO₂R⁵,
or
- a perfluorinated C₁₋₄ alkyl group,
and
(b) at least one reactive compound selected from
(b1) reactive carbonyl compounds selected from the group of aromatic, heteroaromatic or unsaturated aldehydes or ketones, dialdehydes or diketones or acetals, semiaminals or imine derivatives of such reactive carbonyl compounds and/or
(b2) CH-active compounds corresponding to formula (2) or (3): 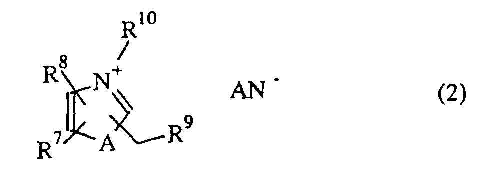
in which R¹⁰ is a C₁₋₁₀ alkyl group, a C₂₋₄ alkenyl group, a C₂₋₄ hydroxyalkyl group, a C₂₋₄ carboxyalkyl group, a C₂₋₄ sulfoalkyl group or an aralkyl group,
R⁷ and R⁸ independently of one another represent a hydrogen atom, a C₁₋₄ alkyl group, a halogen atom, a hydroxy group, a C₁₋₄ alkoxy group or a nitro group or together form a fused aromatic ring,
R⁹ is a hydrogen atom, a C₁₋₄ alkyl group or an aryl group,
A is an oxygen or a sulfur atom, the group -CH=CH- or >N-R¹¹, where R¹¹ is a C₁₋₄ alkyl group, a C₂₋₄ carboxyalkyl group, a C₂₋₄ sulfoalkyl group, a C₂₋₄ sulfoxyalkyl group, a C₂₋₄ hydroxyalkyl group or an aralkyl group, and
AN⁻ is an anion selected from halide, C₁₋₄ alkyl sulfate, C₁₋₄ alkanesulfonate, arenesulfonate, C₁₋₄ perfluoroalkane-sulfonate, tetrafluoroborate, perhalogenate, sulfate, hydrogen sulfate or carboxylate, 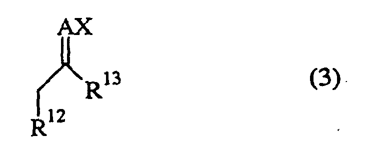 in which R¹² is a C₁₋₄ acyl group, a C₁₋₄ alkylsulfonyl group, a C₁₋₄alkylsulfinyl group, a C₁₋₄ alkylamino group, a di-C₁₋₄-alkyamino group, a vinyl carbonyl group, a methineimino group, a nitrile group, an ester or carboxylic acid amide group which may optionally be substituted by a C₁₋₄ alkyl group, a C₂₋₄ hydroxyalkyl group or an aryl group, and R¹³ represents a C₁₋₄ acyl group, a C₁₋₄ alkoxy group, a C₁₋₄ alkylamino group, a C₁₋₄ acylamino group or a di-C₁₋₄-alkylamino group; the substituents R¹² and R¹³ together with the rest of the molecule may also form a 5-, 6- or 7-membered ring system from the series of thiazolidine-2,5-diones, thiazolidine-2-thion-5-ones, perhydropyrimidine-2,4,6-triones, perhydropyrimidine-2-thione-4,6-diones, cyclopentane-1,3-diones, cyclohexane-1,3-diones, indane-1,3-diones, 2-pyrazoline-5-ones, 1,2-dihydro-6-hydroxy-2-hydroxypyridines or enol esters thereof, and AX represents oxygen, sulfur or a dicyanomethylene group.

2. A composition as claimed in claim 1, **characterized in that** Y is hydrogen.

3. A composition as claimed in claim 1 or 2, **characterized in that** R¹ is hydrogen.

4. A composition as claimed in any of claims 1 to 3, **characterized in that** R⁴ is hydrogen.

5. A composition as claimed in any of claims 1 to 4, **characterized in that** X is an oxygen atom.

6. A composition as claimed in claim 5, **characterized in that** the benzo[b]furan-3-one derivative corresponding to formula (I) is selected from 5-carboxymethoxybenzo[b]furan-3-one, 5-hydroxybenzo[b]furan-3-one or 5-(2'-hydroxyethoxy)-benzo[b]furan-3-one.

7. A composition as claimed in any of claims 1 to 4, **characterized in that** X is a sulfur atom.

8. A composition as claimed in claim 7, **characterized in that** the benzo[b]thiophen-3-one derivative corresponding to formula (I) is selected from 5-hydroxybenzo[b]thiophen-3-one, 5-carboxymethoxybenzo[b]thiophen-3-one, 5,6-dihydroxybenzo[b]thiophen-3-one or 6-hydroxy-5-methoxybenzo[b]thiophen-3-one.

9. A composition as claimed in any of claims 1 to 8, **characterized in that** it contains at least one reactive carbonyl compound of the isatin and isatin derivative type.

10. A composition as claimed in any of claims 1 to 9, **characterized in that** it contains at least one reactive carbonyl compound of the aromatic aldehyde and aromatic aldehyde derivative type.

11. A composition as claimed in claim 9 or 10, **characterized in that** the reactive carbonyl compound is selected from 4-N,N-dimethylaminobenzaldehyde, 4-N,N-dimethylaminocinnamaldehyde, vanillin, 4-N,N-dimethylamino-1-naphthaldehyde and isatin.

12. A composition as claimed in any of claims 1 to 11, **characterized in that** it additionally contains at least one oxidation dye precursor of the primary intermediate and/or secondary intermediate type.

13. A composition as claimed in any of claims 1 to 12, **characterized in that** it additionally contains at least one dye precursor of the indole or indoline type.

14. A composition as claimed in any of claims 1 to 13, **characterized in that** it additionally contains at least one substantive dye.

15. A composition as claimed in any of claims 1 to 14, **characterized in that** it contains an oxidizing agent, preferably hydrogen peroxide.

16. The use of the composition claimed in any of claims 1 to 15 for coloring keratinous fibers.

17. A process for coloring keratinous fibers in which the fibers are treated with the composition claimed in any of claims 1 to 16 and then rinsed.

## Revendications

1. Agent pour la teinture de fibres kératiniques, contenant dans un milieu cosmétiquement acceptable, comme précurseurs de colorant,
(a) au moins un dérivé de la benzo[b]furann-3-one et/ou au moins un dérivé de la benzo[b]thiophén-3-one de formule (1), 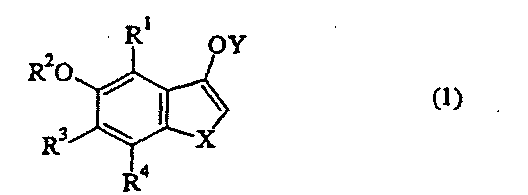 dans laquelle
X représente un atome de soufre ou un atome d'oxygène,
Y représente un atome d'hydrogène, un groupe alkyle en C₁ à C₄ ou un radical acyle en C₁ à C₄ et les radicaux R¹, R³ et R⁴ représentent, indépendamment l'un de l'autre
- un atome d'hydrogène,
- un groupe alkyle en C₁ à C₄, qui peut éventuellement être substitué par
- un ou plusieurs groupes hydroxy
- un groupe amino, éventuellement substitué,
- un groupe carboxy, éventuellement estérifié par un groupe alkyle en C₁ à C₄ ou
- un groupe -SO₂R⁵, où R⁵ représente un groupe hydroxy, un groupe amino éventuellement substitué ou un groupe alkyle en C₁ à C₄,
- un groupe amino éventuellement substitué,
- un groupe alkyle en C₁ à C₄, perfluoré,
- un groupe cyano,
- un groupe alcényle en C₂ à C₅,
- un groupe nitro,
- un atome d'halogène,
- un groupe mercapto éventuellement substitué par un groupe alkyle en C₁ à C₄,
- un groupe -SO₂R⁵ ou
- un groupe -OR⁶,
- un groupe carboxyle éventuellement estérifié ou amidé et R² et, le cas échéant R⁶, représentent indépendamment l'un de l'autre
- un atome d'hydrogène,
- un groupe alkyle en C₁ à C₄ qui peut éventuellement être substitué par
- un ou plusieurs groupes hydroxy
- un groupe amino, éventuellement substitué,
- un groupe carboxy, éventuellement estérifié par un groupe alkyle en C₁ à C₄, ou
- un groupe -SO₂R⁵,
ou
- un groupe alkyle en C₁ à C₄ perfluoré,
et
(b) au moins un composé réactif, choisi parmi
(b1) les composés réactifs carbonyle, choisis dans le groupe des aldéhydes ou cétones aromatiques, hétéroaromatiques ou insaturés, des dialdéhydes ou dicétones ou des acétals, semiaminals ou dérivés imine de ces composés carbonyle réactifs et/ou
(b2) des composés à groupe CH actif, présentant les formules (2) ou (3), 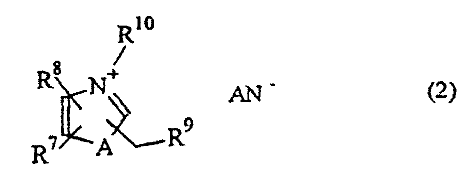
dans laquelle R¹⁰ représente un groupe alkyle en C₁ à C₁₀, un groupe alcényle en C₂ à C₄, un groupe hydroxyalkyle en C₂ à C₄, un groupe carboxyalkyle en C₂ à C₄, un groupe sulfoalkyle en C₂ à C₄ ou un groupe aralkyle,
R⁷ et R⁸ signifient indépendamment l'un de l'autre un atome d'hydrogène, un groupe alkyle en C₁ à C₄, un atome d'halogène, un groupe hydroxy, un groupe alcoxy en C₁ à C₄ ou un groupe nitro ou forment ensemble un cycle aromatique condensé,
R⁹ représente un atome d'hydrogène, un groupe alkyle en C₁ à C₄ ou un groupe aryle,
A représente un atome d'oxygène ou un atome de soufre, le groupe -CH=CH- ou >N-R¹¹, où R¹¹ représente un groupe alkyle en C₁ à C₄, un groupe carboxyalkyle en C₂ à C₄, un groupe sulfoalkyle en C₂ à C₄, un groupe sulfoxyalkyle en C₂ à C₄, un groupe hydroxyalkyle en C₂ à C₄ ou un groupe aralkyle et
AN⁻ représente un anion, choisi parmi halogénure, alkylsulfate en C₁ à C₄, alcanesulfonate en C₁ à C₄, arylènesulfonate, perfluoroalcanesulfate en C₁ à C₄, tétrafluoroborate, perhalogénate, sulfate, hydrogénosulfate ou carboxylate, 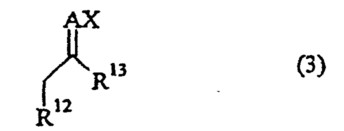 où R¹² représente un groupe acyle en C₁ à C₄, un groupe alkylsulfonyle en C₁ à C₄, un groupe alkylsulfinyle en C₁ à C₄, un groupe alkylamino en C₁ à C₄, un groupe dialkylamino en C₁ à C₄, un groupe vinylcarbonyle, un groupe méthinimino, un groupe nitrile, un groupe ester ou amide d'acide carboxylique, qui peuvent éventuellement être substitués par un groupe alkyle en C₁ à C₄, un groupe hydroxyalkyle en C₂ à C₄ ou un groupe aryle, et
R¹³ représente un groupe acyle en C₁ à C₄, un groupe alcoxy en C₁ à C₄, un groupe alkylamino en C₁ à C₄, un groupe acylamino en C₁ à C₄ ou un groupe dialkylamino en C₁ à C₄,
les radicaux R¹² et R¹³ pouvant également former avec la molécule résiduelle un système cyclique à 5, 6 ou 7 chaînons de la série constituée par les thiazolidine-2,5-diones, les thiazolidine-2-thion-5-ones, les perhydropyrimidine-2,4,6-triones, les perhydropyrimidine-2-thione-4,6-diones, les cyclopentane-1,3-diones, les cyclohexane-1,3-diones, les indane-1,3-diones, les 2-pyrazolin-5-ones, les 1,2-dihydro-6-hydroxy-2-hydroxypyridines ou leurs énolesters et
AX représente un atome d'oxygène, un atome de souffre ou un groupe dicyanométhylène.

2. Agent selon la revendication 1, **caractérisé en ce que** Y représente un atome d'hydrogène.

3. Agent selon l'une quelconque des revendications 1 ou 2, **caractérisé en ce que** R¹ représente un atome d'hydrogène.

4. Agent selon l'une quelconque des revendications 1 à 3, **caractérisé en ce que** R⁴ représente un atome d'hydrogène.

5. Agent selon l'une quelconque des revendications 1 à 4, **caractérisé en ce que** X représente un atome d'oxygène.

6. Agent selon la revendication 5, **caractérisé en ce que** le dérivé de benzo[b]furann-3-one de formule (1) est choisi parmi la 5-carboxyméthoxy-benzo[b]furann-3-one, la 5-hydroxybenzo[b]furann-3-one ou la 5-(2'-hydroxyéthoxy)benzo[b]furann-3-one.

7. Agent selon l'une quelconque des revendications 1 à 4, **caractérisé en ce que** X représente un atome de souffre.

8. Agent selon la revendication 7, **caractérisé en ce que** le dérivé de benzo[b]thiophén-3-one de formule (1) est choisi parmi la 5-hydroxybenzo[b]thiophén-3-one, la 5-carboxyméthoxybenzo[b]thiophén-3-one, la 5,6-dihydroxybenzo[b]thiophén-3-one ou la 6-hydroxy-5-méthoxybenzo [b]thiophén-3-one.

9. Agent selon l'une quelconque des revendications 1 à 8, **caractérisé en ce qu'**il contient au moins un composé carbonyle réactif de type de l'isatine et ses dérivés.

10. Agent selon l'une quelconque des revendications 1 à 9, **caractérisé en ce qu'**il contient au moins un composé carbonyle réactif du type des aldéhydes aromatiques et leurs dérivés.

11. Agent selon l'une quelconque des revendications 9 ou 10, **caractérisé en ce que** le composé carbonyle réactif est choisi parmi le 4-NN-diméthylamino-benzaldéhyde, l'aldéhyde 4-N,N-diméthylaminocinnamique, la vanilline, le 4-N,N-diméthylamino-1-naphtaldéhyde et l'isatine.

12. Agent selon l'une quelconque des revendications 1 à 11, **caractérisé en ce qu'**il contient en outre au moins un précurseur d'un colorant d'oxydation du type agent de développement et/ou agent de couplage.

13. Agent selon l'une quelconque des revendications 1 à 12, **caractérisé en ce qu'**il contient en outre au moins un précurseur de colorant du type indole ou indoline.

14. Agent selon l'une quelconque des revendications 1 à 13, **caractérisé en ce qu'**il contient en outre au moins un colorant montant directement sur la fibre.

15. Agent selon l'une quelconque des revendications 1 à 14, **caractérisé en ce qu'**il contient un oxydant, de préférence du peroxyde d'hydrogène.

16. Utilisation d'un agent selon l'une quelconque des revendications 1 à 15 pour la teinture de fibres kératiniques.

17. Procédé pour la teinture de fibres kératiniques, dans lequel les fibres sont traitées avec un agent selon l'une quelconque des revendications 1 à 16 et sont ensuite rincées.
